(19) Europäisches Patentamt
European Patent Office
Office européen des brevets

(11) EP 4 146 480 B1

(12) # EUROPÄISCHE PATENTSCHRIFT

(45) Veröffentlichungstag und Bekanntmachung des Hinweises auf die Patenterteilung:
**29.05.2024 Patentblatt 2024/22**

(21) Anmeldenummer: **21715592.8**

(22) Anmeldetag: **29.03.2021**

(51) Internationale Patentklassifikation (IPC):
***B41M 5/333*** *(2006.01)* ***C07C 309/76*** *(2006.01)*
***B41M 5/327*** *(2006.01)* ***B41M 5/337*** *(2006.01)*

(52) Gemeinsame Patentklassifikation (CPC):
**B41M 5/3333; C07C 309/76;** B41M 5/3275;
B41M 5/3375

(86) Internationale Anmeldenummer:
**PCT/EP2021/058119**

(87) Internationale Veröffentlichungsnummer:
**WO 2021/223939 (11.11.2021 Gazette 2021/45)**

(54) **WÄRMEEMPFINDLICHES AUFZEICHNUNGSMATERIAL**

HEAT-SENSITIVE RECORDING MATERIAL

MATÉRIAU D'IMPRESSION THERMOSENSIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.05.2020 DE 102020112411**

(43) Veröffentlichungstag der Anmeldung:
**15.03.2023 Patentblatt 2023/11**

(73) Patentinhaber: **Koehler Innovation & Technology GmbH**
**77704 Oberkirch (DE)**

(72) Erfinder:
- **STALLING, Timo**
  **77767 Appenweier (DE)**
- **HORN, Michael**
  **77654 Offenburg (DE)**

(74) Vertreter: **Meissner Bolte Partnerschaft mbB**
**Patentanwälte Rechtsanwälte**
**Postfach 86 06 24**
**81633 München (DE)**

(56) Entgegenhaltungen:
**EP-A1- 2 923 851 WO-A1-2017/111032**

- **Anonymous: "Registry - RN 406713-68-6, entered STN 23.04.2002 - Benzenesulfonic acid 4- chloro-3 - [[[(4- fluorophenyl)amino]carbonyl]amino] - 4-[[[(4-fluorophenyl)amino]carbonyl]amino]phenyl ester", Registry , 23. April 2002 (2002-04-23), XP055812828, Gefunden im Internet: URL:none [gefunden am 2021-06-10]**
- **Anonymous: "Reaxys - 4-({[(4-fluorophenyl)amino]carbonyl}amino) phenyl 4-chloro-3-({[(4-fluorophenyl)amino]carbonyl}amino)benzenesulfonate", Reaxys Database , 10. Juni 2021 (2021-06-10), XP055812827, Gefunden im Internet: URL:none [gefunden am 2021-06-10]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 146 480 B1

## Beschreibung

Die Erfindung betrifft einen Farbentwickler, ein wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, sowie ein Verfahren zu dessen Herstellung.

Wärmeempfindliche Aufzeichnungsmaterialien (Thermopapiere) für die Thermodirekt-Druckanwendung, die eine auf einem Trägersubstrat aufgebrachte wärmeempfindliche farbbildende Schicht (Thermoreaktionsschicht) aufweisen, sind seit langem bekannt.

In der wärmeempfindlichen farbbildenden Schicht liegen üblicherweise ein Farbbildner und ein Farbentwickler vor, die unter Wärmeeinwirkung miteinander reagieren und so zu einer Farbentwicklung führen. Weit verbreitet sind preisgünstige (bis)phenolische Farbentwickler, wie beispielsweise Bisphenol A und Bisphenol S, mit welchen wärmeempfindliche Aufzeichnungsmaterialien erhalten werden können, die für zahlreiche Anwendungen ein akzeptables Leistungsprofil aufweisen. Ebenfalls bekannt sind wärmeempfindliche Aufzeichnungsmaterialien, die in der wärmeempfindlichen farbbildenden Schicht einen nicht-phenolischen Farbentwickler enthalten. Diese wurden entwickelt, um die Beständigkeit des Schriftbildes zu verbessern, insbesondere auch dann, wenn das bedruckte wärmeempfindliche Aufzeichnungsmaterial über längere Zeit bei höheren Temperaturen und/oder Luftfeuchtigkeit gelagert werden soll. Insbesondere vor dem Hintergrund der öffentlichen Diskussionen über das toxische Potenzial (bis)phenolischer Chemikalien ist das Interesse an nicht-phenolischen Farbentwicklern stark angestiegen. Hierbei war es Ziel, die Nachteile der (bis)phenolischen Farbentwickler zu vermeiden, allerdings sollten die technischen Leistungseigenschaften, die mit phenolischen Farbentwicklern erzielt werden können, zumindest beibehalten, vorzugsweise aber verbessert werden.

Der Stand der Technik zu nicht-phenolischen Farbentwicklern lässt trotz der großen chemischen Diversität dieser Stoffe gemeinsame strukturelle Merkmale erkennen.

So ist eine 1,3-disubstituierte (Thio)Ureido-Substruktur (Y-NH-C(X)-NH-Z mit X = S oder O) ein gemeinsames Merkmal zahlreicher nicht-phenolischer Farbentwickler. Durch passende Wahl der Gruppen Y und Z können die für die Eignung als Farbentwickler relevanten funktionellen Eigenschaften moduliert werden.

Große Verbreitung haben Farbentwickler mit Sulfonyl-Harnstoff-Strukturen (-SOz-NH-CO-NH-) erfahren, da sie relativ leicht herstellbar sind und die mit ihnen hergestellten wärmeempfindlichen Aufzeichnungsmaterialien gute anwendungstechnische Eigenschaften aufweisen.

Die EP 0 526 072 A1 und die EP 0 620 122 B1 offenbaren Farbentwickler aus der Klasse der aromatischen Sulfonyl-(Thio)Harnstoffe. Mit diesen können wärmeempfindliche Aufzeichnungsmaterialien erhalten werden, die sich durch eine relativ hohe Bildbeständigkeit auszeichnen. Ferner weisen die auf diesen Farbentwicklern basierenden wärmeempfindlichen Aufzeichnungsmaterialien eine brauchbare thermische Empfindlichkeit bei guter Oberflächenweiße auf, so dass es bei entsprechender Gestaltung der Rezeptur der wärmeempfindlichen farbbildenden Schicht vergleichsweise leicht möglich ist, hohe Druckdichten unter Verwendung handelsüblicher Thermodrucker zu erzeugen.

Die WO 0 035 679 A1 offenbart aromatische und heteroaromatische Sulfonyl-(Thio)harnstoffverbindungen (X = S oder O) und/oder Sulfonyl-Guanidine (X = NH) der Formel Ar'-$SO_2$-NH-C(X)-NH-Ar, wobei Ar durch eine zweiwertige Linkergruppe an weitere aromatische Gruppen geknüpft ist. Ein in der Praxis weit verbreiteter nicht-phenolischer Farbentwickler aus dieser Klasse, 4-Methyl-N-(((3-(((4-methylphenyl)sulfonyl)oxy)phenyl)amino)carbonyl)benzolsulfonamid (Handelsname Pergafast 201®, BASF), zeichnet sich durch die Ausgewogenheit der anwendungstechnischen Eigenschaften der mit diesem Farbentwickler hergestellten wärmeempfindlichen Aufzeichnungsmaterialien aus. Insbesondere besitzen diese eine gute dynamische Ansprechempfindlichkeit und eine im Vergleich zu mit (bis)phenolischen Farbentwicklern erhaltenen Aufzeichnungsmaterialien höhere Beständigkeit des Ausdrucks bei Lagerung unter harschen Umweltbedingungen oder gegenüber hydrophoben Stoffen.

Sulfonylharnstoffe neigen in Gegenwart von Wasser/Feuchtigkeit und/oder in der Wärme zu hydrolytische Zersetzungsreaktionen (M. Eckhardt, T.J. Simat, Chemosphere, 186, 1016 (2017)). Dies führt dazu, dass wärmeempfindliche Aufzeichnungsmaterialien bei Lagerung im unbedruckten Zustand unter Bedingungen erhöhter Luftfeuchtigkeit und/oder Temperatur eine teilweise Zersetzung des Farbentwicklers erfahren können.

Da die Schreibleistung (dynamische Ansprechempfindlichkeit) wärmeempfindlicher Aufzeichnungsmaterialien von der Menge des in der wärmeempfindlichen Schicht vorliegenden Farbentwicklers abhängt, verliert ein derart über längere Zeiträume gelagertes wärmeempfindliches Aufzeichnungsmaterial einen Teil des Farbentwicklers und büßt dadurch teilweise seine Schreibleistung ein.

Die oben angesprochene Möglichkeit der Modulation der Eigenschaften 1,3-disubstituierte (Thio)Ureido-Substruktur kann auch durch Einbezug von zur Ureido-Einheit in vorteilhafter konjugativer Verbundenheit stehender Struktureinheiten erreicht werden.

Ein solcher Ansatz wurde z.B. in der EP 2 923 851 A1 verfolgt. So offenbart die EP 2 923 851 A1 Farbentwickler der allgemeinen Formel $R^1$-NH-CO-NH-Ar-NH-$SO_2$-$R^2$, wobei $R^1$, $R^2$ und Ar (un)substituierte Aryl-Reste sein können. Ein in der Praxis weit verbreiteter nicht-phenolischer Farbentwickler aus dieser Klasse ist N-(2-(3-Phenylureido)phenyl)benzolsulfonamid (Handelsname NKK 1304®, Nippon Soda Co. Ltd.). Obwohl mit den auf diesen Farbentwicklern

basierenden wärmeempfindlichen Aufzeichnungsmaterialien eine gute dynamische Sensitivität gewährleistet werden kann, ist die Stabilität des Farbkomplexes - insbesondere gegenüber Weichmachern oder Klebstoffen - bescheiden.

Ein vergleichbares Konzept wurde auch in der WO 2017 111032 A1 realisiert, die Farbentwickler der allgemeinen Formel Ph-NH-CO-NH-$C_6H_4$-O-$SO_2$-Ar, wobei Ar ein (un)substituierter Phenylrest sein kann, offenbart.

Schon frühzeitig wurde versucht, die Leistungsfähigkeit nicht-phenolischer Farbentwickler durch Verwendung von Farbentwicklerstrukturen, die mehr als nur eine der für den Farbbildungsprozess relevanten Struktureinheiten enthalten, zu verbessern.

Die JP H 06 227 142 A offenbart Farbentwickler mit zwei, drei oder mehr (Thio)Harnstoffeinheiten (Bis-, Tris-, Polykis-Harnstoffe) in der Verbindung $Ar^1$-NH-C(X)-$NH)_n$-A, die an eine meist aromatische Einheit A gebunden sind (X = S oder O). Strukturell ähnlich aufgebaut sind die in der EP 633 145 A1, der JP H 0 821 109 A, der JP H 08 244 355 A und der JP H 11 268 422 A beschriebenen Farbentwickler.

Obwohl Bis- oder Polykis-Harnstoffderivate gute Wasserstoffbrücken-Akzeptor- und Donator-Eigenschaften besitzen und daher geeignet sind, den Farbkomplex zu stabilisieren, bedingen die sich zwischen den einzelnen Harnstoffeinheiten ausbildenden Wasserstoffbrücken-Netzwerke einen relativ hohen Schmelzpunkt und eine geringe Löslichkeit dieser Stoffe in typischen thermischen Lösungsmitteln aus der wärmeempfindlichen Schicht mit der Folge, dass die thermische Ansprechempfindlichkeit (sog. dynamische Sensitivität) der mit diesen Farbentwicklern hergestellten wärmeempfindlichen Aufzeichnungsmaterialien zu wünschen übrig lässt.

Aufgabe der vorliegenden Erfindung ist es daher, vorstehend geschilderte Nachteile des Standes der Technik zu beheben. Insbesondere besteht die Aufgabe der vorliegenden Erfindung darin, einen Farbentwickler und ein diesen enthaltendes wärmeempfindliches Aufzeichnungsmaterial bereitzustellen, welches ein ausgewogenes anwendungstechnisches Eigenschaftsprofil aufweist und eine praxistaugliche Druckdichte erreicht, das mit wärmeempfindlichen Aufzeichnungsmaterialien, die auf bekannten nicht-phenolischen Farbentwicklerstoffen beruhen, vergleichbar ist, dabei aber eine hohe Beständigkeit des Druckbildes, insbesondere beim Inkontaktbringen der wärmeempfindlichen Schicht mit hydrophoben Stoffen wie Weichmacher aus Folienmaterialien, Ölen, Fetten u. dgl., aufweist, vorzugsweise ohne auf spezielle Rezepturbestandteile in der wärmeempfindlichen Funktionsschicht, wie Alterungsschutzmittel oder spezielle Schmelzhilfsmittel mit eingeschränkter Verfügbarkeit und hohem Preis, angewiesen zu sein. Eine weitere Aufgabe der vorliegenden Erfindung ist es, Farbentwickler bzw. ein wärmeempfindliches Aufzeichnungsmaterial zur Verfügung zu stellen, welches in der Lage ist, die anwendungstechnischen notwendigen funktionellen Eigenschaften (insbesondere die thermische Ansprechempfindlichkeit) auch bei Lagerung über längere Zeiträume und/oder unter extremen Klimabedingungen (hohe Luftfeuchtigkeit und/oder Temperatur) des unbedruckten wärmeempflindlichen Aufzeichnungsmaterials zu gewährleisten.

Erfindungsgemäß werden diese Aufgaben durch den Einsatz einer Verbindung nach Anspruch 1 in einem wärmeempfindlichen Aufzeichnungsmaterial nach Anspruch 10 gelöst.

Überraschenderweise hat es sich gezeigt, dass es möglich ist, mit Farbentwicklern aus der erfindungsgemäßen Klasse (I) wärmeempfindliche Aufzeichnungsmaterialien zu erhalten, welche sich durch eine hervorragende Beständigkeit des Schriftbilds auszeichnen, insbesondere wenn das Druckbild hydrophoben Stoffen wie Weichmachern ausgesetzt wird. Die mit den erfindungsgemäßen Farbentwicklern hergestellten Aufzeichnungsmaterialien weisen auch eine ausgezeichnete Langzeitlagerfähigkeit auf. So leidet das im Thermodrucker entwickelte Schriftbild selbst nach Lagerung im unbedruckten (weißen) Zustand über mehrere Wochen bei hoher Umgebungsfeuchte und/oder hohen Temperaturen kaum.

Die Verbindung nach Anspruch 1 besitzt die Formel (I),

$$Ar^1\text{-NH-CO-NH-}C_6H_4\text{-}SO_2\text{-O-}C_6H_4\text{-NH-CO-NH-}Ar^2 \qquad (I)$$

wobei $Ar^1$ und $Ar^2$ unabhängig voneinander ein unsubstituierter oder substituierter Phenyl-, Naphthyl- und/oder Heteroaryl-Rest sind.

Vorzugsweise ist $Ar^1$ ein Phenyl-Rest.

Vorzugsweise ist $Ar^2$ ein Phenyl-Rest.

Bevorzugt ist $Ar^1$ mit mindestens einem $C_1$-$C_5$-Alkyl-, einem Alkenyl-, einem Alkinyl-, einem Benzyl-, einem Formyl-, einem Halogen-, einem NOz-, einem CN-, einem R-CO-, einem RO-, einem ROzC-, einem R-OCO-, einem R-SOz-O-, einem R-O-SOz-, einem R-SOz-NH-, einem R-NH-SOz-, einem R-NH-CO- oder einem R-CO-NH-Rest substituiert, wobei Rein $C_1$-$C_5$-Alkyl-, ein Alkenyl-, ein Alkinyl-, ein Phenyl-, ein Tolyl- oder ein Benzyl-Rest ist.

Besonders bevorzugt ist $Ar^1$ mit mindestens einem $C_1$-$C_5$-Alkyl-, einem Halogen-, einem NOz-, einem CN-, einem R-CO-, einem RO- oder einem ROzC-Rest, wobei R ein $C_1$-$C_5$-Alkyl-Rest ist, substituiert.

Vorzugsweise ist $Ar^1$ einfach substituiert.

Vorzugsweise ist $Ar^1$ ein einfach substituierter Phenyl-Rest.

Bevorzugt ist $Ar^2$ mit mindestens einem $C_1$-$C_5$-Alkyl-, einem Alkenyl-, einem Alkinyl-, einem Benzyl-, einem Formyl-, einem Halogen-, einem NOz-, einem CN-, einem R-CO-, einem RO-, einem ROzC-, einem R-OCO-, einem R-SOz-O-, einem R-O-SOz-, einem R-SOz-NH-, einem R-NH-SOz-, einem R-NH-CO- oder einem R-CO-NH-Rest substituiert, wobei Rein $C_1$-$C_5$-Alkyl-, ein Alkenyl-, ein Alkinyl-, ein Phenyl-, ein Tolyl- oder ein Benzyl-Rest ist.

Besonders bevorzugt ist $Ar^2$ mit mindestens einem $C_1$-$C_5$-Alkyl-, einem Halogen-, einem $NO_2$-, einem CN-, einem R-CO-, einem RO- oder einem ROzC-Rest, wobei R ein $C_1$-$C_5$-Alkyl-Rest ist, substituiert.

Vorzugsweise ist $Ar^2$ einfach substituiert.

Vorzugsweise ist $Ar^2$ ein einfach substituierter Phenyl-Rest.

Die Substitution von $Ar^1$ und/oder $Ar^2$ mit mindestens einem $C_1$-$C_5$-Alkyl-Rest erfolgt vorzugsweise derart, dass der $C_1$-$C_5$-Alkyl-Rest ein Methyl- oder ButylRest, besonders bevorzugt ein Methyl-Rest, ist.

Die Substitution von $Ar^1$ und/oder $Ar^2$ mit mindestens einem Halogen-Rest erfolgt vorzugsweise derart, dass der Halogen-Rest ein Chlorid-Rest und/oder ein Fluorid-Rest ist.

Die Substitution von $Ar^1$ und/oder $Ar^2$ mit mindestens einem RO-Rest erfolgt vorzugsweise derart, dass der RO-Rest ein $CH_3O$-Rest ist.

Die Substitution von $Ar^1$ und/oder $Ar^2$ mit mindestens einem R-CO-Rest erfolgt vorzugsweise derart, dass der R-CO-Rest ein $CH_3$-CO-Rest ist.

In einer besonders bevorzugten Ausführungsform ist sowohl $Ar^1$ als auch $Ar^2$ ein Phenyl-Rest. Derartige Verbindungen sind relativ einfach und kostengünstig herzustellen und liefern hinsichtlich der nachstehend beschriebenen Eigenschaften gute Ergebnisse.

In einer besonders bevorzugten Ausführungsform ist der $Ar^1$-NH-CO-NH-Rest und der $Ar^2$-NH-CO-NH-Rest in 2- bzw. 3'-, in 2- bzw. 4'-, in 3- bzw. 2'-, in 3- bzw. 3'-, in 3- bzw. 4'-, in 4- bzw. 2'-, in 4- bzw. 3'- oder in 4- bzw. 4'-Stellung zu der -$C_6H_4$-$SO_2$-O-$C_6H_4$-Gruppe angeordnet.

Besonders bevorzugt ist die Anordnung in 3- bzw. 2'- oder 4- bzw. 3'-Stellung, da derartige Verbindungen relativ leicht herstellbar sind und gute Eigenschaften zeigen.

Besonders bevorzugte Verbindungen der Formel (I) sind in folgender Tabelle 1 dargestellt:

Tabelle 1: Bevorzugte Verbindungen der Formel (I) mit den angegebenen Bedeutungen für die Anordnung der $Ar^1$-NH-CO-NH- und der $Ar^2$-NH-CO-NH-Gruppen sowie der angegebenen Bedeutung von $Ar^1$ und $Ar^2$ (R wie vorstehend erwähnt).

| Anordnung von $Ar^1$-NH-CO-NH- und $Ar^2$-NH-CO-NH- -an der -$C_6H_4$-$SO_2$-O-$C_6H_4$-Gruppe | $Ar^1$ | $Ar^2$ |
|---|---|---|
| 2,3' | Phenyl- | Phenyl- |
| 2,4' | Phenyl- | Phenyl- |
| 3,2' | Phenyl- | Phenyl- |
| 3,3' | Phenyl- | Phenyl- |
| 3,4' | Phenyl- | Phenyl- |
| 4,2' | Phenyl- | Phenyl- |
| 4,3' | Phenyl- | Phenyl- |
| 4,4' | Phenyl- | Phenyl- |
| 4,3' | $C_1$-$C_5$-Alkyl substituierter Phenyl- | $C_1$-$C_5$-Alkyl substituierter Phenyl- |
| 4,3' | 1-Naphthyl- | 1-Naphthyl- |
| 4,3' | RO-substituierter Phenyl- | RO-substituierter Phenyl- |
| 4,3' | Halogensubstituierter Phenyl- | Halogensubstituierter Phenyl- |
| 4,3' | RO-substituierter Phenyl- | RO-substituierter Phenyl- |
| 4,3' | $RO_2C$-substituierter Phenyl- | $RO_2C$-substituierter Phenyl- |
| 4,3' | CN-substituierter Phenyl- | CN-substituierter Phenyl- |
| 4,3' | Nitrosubstituierter Phenyl- | Nitro-substituierter Phenyl- |
| 4,3' | RO-substituierter Phenyl- | Nitro-substituierter Phenyl- |
| 4,3' | Nitrosubstituierter Phenyl- | RO-substituierter Phenyl- |

Die Herstellung der erfindungsgemäßen Verbindung der Formel (I) kann nach an sich bekannten Methoden erfolgen.

Die Reaktionsschemata 1 bis 3 veranschaulichen einen möglichen Syntheseweg für die erfindungsgemäße Verbindung der Formel (I) am Beispiel der Verbindungen gemäß Tabelle 1.

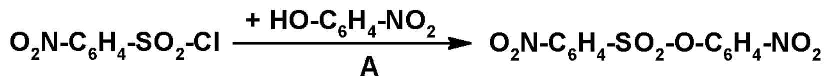


Reaktionsschema 1

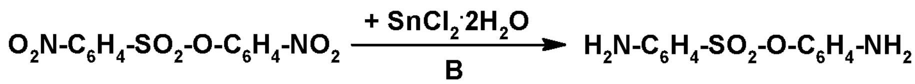


Die im Zusammenhang mit der Verbindung der Formel (I) aufgeführten bevorzugten Ausführungsformen gelten ebenfalls für das Verfahren zu dessen Herstellung.

Wie erwähnt, betrifft die vorliegende Erfindung auch ein wärmeempfindliches Aufzeichnungsmaterial, umfassend ein Trägersubstrat sowie eine mindestens einen Farbbildner und einen phenolfreien Farbentwickler enthaltende wärmeempfindliche farbbildende Schicht, wobei der mindestens eine phenolfreie Farbentwickler die Verbindung der vorstehenden beschriebenen Formel (I) ist.

Die Verbindung der Formel (I) liegt vorzugsweise in einer Menge von etwa 3 bis etwa 35 Gew.-%, besonders bevorzugt in einer Menge von etwa 10 bis etwa 25 Gew.-%, bezogen auf den gesamten Feststoffgehalt der wärmeempfindlichen Schicht, vor.

Die Auswahl des Trägersubstrates ist nicht kritisch. Allerdings ist es bevorzugt, als Trägersubstrat Papier, synthetisches Papier und/oder eine Kunststofffolie einzusetzen.

Gegebenenfalls liegt zwischen dem Trägersubstrat und der wärmeempfindlichen Schicht mindestens eine weitere Zwischenschicht vor, wobei dieser Zwischenschicht die Aufgabe zukommt, die Oberflächenglätte des Trägers für die wärmeempfindliche Schicht zu verbessern und eine Wärmebarriere zwischen dem Trägerpapier und der wärmeempfindlichen Schicht zu gewährleisten. Vorzugsweise kommen in dieser Zwischenschicht organische Hohlkugelpigmente und/oder kalzinierte Kaoline zum Einsatz.

Auch kann mindestens eine Schutzschicht und/oder mindestens eine die Bedruckbarkeit begünstigende Schicht im erfindungsgemäßen wärmeempfindlichen Aufzeichnungsmaterial vorliegen, wobei diese Schichten auf der Vorder- oder Rückseite des Substrats aufgebracht werden können.

Hinsichtlich der Wahl des Farbbildners unterliegt die vorliegende Erfindung ebenfalls keinen wesentlichen Einschränkungen. Bevorzugt ist der Farbbildner jedoch ein Farbstoff vom Triphenylmethan-Typ, vom Fluoran-Typ, vom Azaphthalid-Typ und/oder vom Fluoren-Typ. Ein ganz besonders bevorzugter Farbbildner ist ein Farbstoff vom Fluoran-Typ, da er dank der Verfügbarkeit und der ausgewogenen anwendungsbezogenen Eigenschaften die Bereitstellung eines Aufzeichnungsmaterials mit einem attraktiven Preis-Leistungsverhältnis ermöglicht.

Besonders bevorzugte Farbstoffe vom Fluoran-Typ sind:

3-Diethylamino-6-methyl-7-anilinofluoran,
3-(*N*-Ethyl-*N*-p-toludinamino)-6-methyl-7-anilinofluoran,
3-(*N*-Ethyl-*N*-isoamylamino)-6-methyl-7-anilinofluoran,
3-Diethylamino-6-methyl-7-(o,p-dimethylanilino)fluoran,
3-Pyrrolidino-6-methyl-7-anilinofluoran,
3-(Cyclohexyl-*N*-methylamino)-6-methyl-7-anilinofluoran,
3-Diethylamin-7-(m-trifluoromethylanilino)fluoran,
3-*N*-n-Dibutylamin-6-methyl-7-anilinofluoran,
3-Diethylamino-6-methyl-7-(m-methylanilino)fluoran,
3-*N*-n-Dibutylamin-7-(o-chloroanilino) fluoran,
3-(*N*-Ethyl-*N*-tetrahydrofurfurylamin)-6-methyl-7-anilino-fluoran,
3-(*N*-Methyl-*N*-propylamin)-6-methyl-7-anilinofluoran,
3-(*N*-Ethyl-*N*-ethoxypropylamin)-6-methyl-7-anilinofluoran,
3-(*N*-Ethyl-*N*-isobutylamin)-6-methyl-7-anilinofluoran und/oder
3-Dipentylamin-6-methyl-7-anilinofluoran.

Die Farbbildner können als Einzelstoffe als auch als beliebige Gemische zweier oder mehrerer Farbbildner zur Anwendung kommen, vorausgesetzt, die wünschenswerten anwendungstechnischen Eigenschaften der Aufzeichnungsmaterialien leiden darunter nicht.

Der Farbbildner liegt vorzugsweise in einer Menge von etwa 5 bis etwa 30 Gew.- %, besonders bevorzugt in einer Menge von etwa 8 bis etwa 20 Gew.-%, bezogen auf den gesamten Feststoffgehalt der wärmeempfindlichen Schicht, vor.

Zur Steuerung spezieller anwendungstechnischer Eigenschaften kann es vorteilhaft sein, wenn mindestens zwei unter die Formel (I) fallende Verbindungen als Farbentwickler in der wärmeempfindlichen Schicht vorliegen.

Desgleichen können ein oder mehrere weitere (bis)phenolische oder nicht-phenolische Farbentwickler zusätzlich zu den Verbindungen der Formel (I) in der wärmeempfindlichen farbbildenden Schicht vorliegen.

Neben dem mindestens einem Farbbildner und dem mindestens einem Farbentwickler können in der wärmeempfindlichen farbbildenden Schicht ein oder mehrere Sensibilisierungsmittel, auch thermische Lösungsmittel genannt, vorliegen, was den Vorteil hat, dass die Steuerung der thermischen Druckempfindlichkeit leichter zu realisieren ist.

Generell kommen als Sensibilisierungsmittel vorteilhafterweise kristalline Stoffe in Betracht, deren Schmelzpunkt zwischen etwa 90 °C und etwa 150 °C liegt und die im geschmolzenen Zustand die farbbildenden Komponenten (Farbbildner und Farbentwickler) lösen, ohne die Ausbildung des Farbkomplexes zu stören.

Vorzugsweise ist das Sensibilisierungsmittel ein Fettsäureamid, wie Stearamid, Behenamid oder Palmitamid, ein Ethylen-bis-fettsäureamid, wie *N*,*N*'-Ethylen-bis-stearinsäureamid oder *N*,*N*'-Ethylen-bis-ölsäureamid, ein Fettsäurealkanolamid, wie *N*-(Hydroxymethyl)stearamid, *N*-Hydroxymethylpalmitamid oder Hydroxyethylstearamid, ein Wachs, wie Polyethylenwachs oder Montanwachs, ein Carbonsäureester, wie Dimethylterephthalat, Dibenzylterephthalat, Benzyl-4-benzyloxybenzoat, Di-(4-methylbenzyl)oxalat, Di-(4-chlorbenzyl)oxalat oder Di-(4-benzyl)oxalat, Ketone, wie 4-Acetylbiphenyl, ein aromatischer Ether, wie 1,2-Diphenoxy-ethan, 1,2-Di-(3-methylphenoxy)ethan, 2-Benzyloxynaphthalin, 1,2-Bis-(phenoxymethyl)benzol oder 1,4-Diethoxynaphthalin, ein aromatisches Sulfon, wie Diphenylsulfon, und/oder ein aromatisches Sulfonamid, wie 4-Toluolsulfonamid, Benzolsulfonanilid oder N-Benzyl-4-toluolsulfonamid oder aromatische Kohlenwasserstoffe wie, 4-Benzylbiphenyl.

Das Sensibilisierungsmittel liegt vorzugsweise in einer Menge von etwa 10 bis etwa 40 Gew.-%, besonders bevorzugt in einer Menge von etwa 15 bis etwa 25 Gew. %, bezogen auf den gesamten Feststoffgehalt der wärmeempfindlichen Schicht, vor.

In einer weiteren bevorzugten Ausführungsform liegt neben dem Farbbildner, dem phenolfreien Farbentwickler und dem Sensibilisierungsmittel optional mindestens ein Stabilisator (Alterungsschutzmittel) in der wärmeempfindlichen farbbildenden Schicht vor.

Bei dem Stabilisator handelt es sich vorzugsweise um sterisch gehinderte Phenole, besonders bevorzugt um 1,1,3-Tris-(2-methyl-4-hydroxy-5-cyclohexyl- phenyl)-butan, 1,1,3-Tris-(2-methyl-4-hydroxy-5-tert-butylphenyl)-butan, 1,1-Bis-(2-methyl-4-hydroxy-5-tert-butyl-phenyl)-butan.

Auch Harnstoff-Urethan-Verbindungen der allgemeinen Formel (II) (Handelsprodukt UU) oder vom 4,4'-Dihydroxydiphenylsulfon abgeleitete Ether, wie 4-Benzyloxy-4'-(2-methylglycidyloxy)-diphenylsulfon (Handelsname NTZ-95®, Nippon Soda Co. Ltd.), oder oligomere Ether der allgemeinen Formel (III) (Handelsname D90®, Nippon Soda Co. Ltd.), sind als Stabilisatoren im erfindungsgemäßen Aufzeichnungsmaterial einsetzbar.

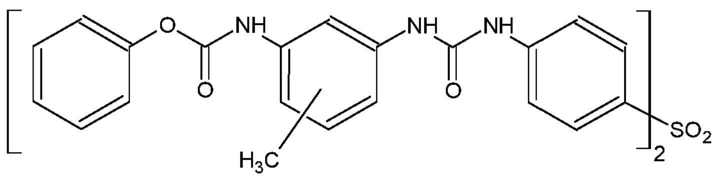

(II)

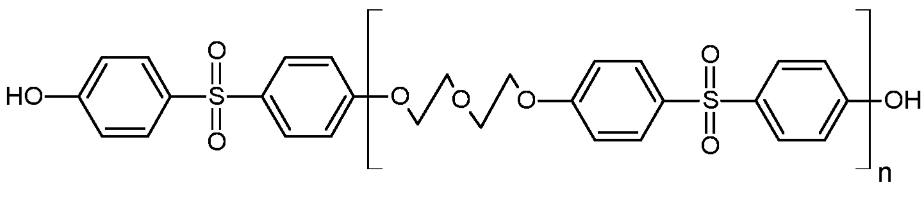

(III)

Besonders bevorzugt sind die Harnstoff-Urethan-Verbindungen der allgemeinen Formel (II).

Der Stabilisator liegt vorzugsweise in einer Menge von 0,2 bis 0,5 Gew.-Teilen, bezogen auf 1 Gew.-Teil des mindestens einen phenolfreien Farbentwicklers der Verbindung der Formel (I), vor.

In einer weiteren bevorzugten Ausführungsform liegt in der wärmeempfindlichen farbbildenden Schicht mindestens ein Bindemittel (Binder) vor. Bei diesem handelt es sich vorzugsweise um wasserlösliche Stärken, Stärkederivate, stärkebasierte Biolatices vom EcoSphere®-Typ, Methylcellulose, Hydroxyethylcellulose, Carboxymethylcellulosen, partiell oder vollständig verseifte Polyvinylalkohole, chemisch modifizierte Polyvinylalkohole oder Styrolmaleinsäureanhydrid-Copolymere, Styrolbutadien-Copolymere, Acrylamid-(Meth)acrylat-Copolymere, Acrylamid-Acrylat-Methacrylat-Terpolymere, Polyacrylate, Poly(meth)-acrylsäureester, Acrylat-Butadien-Copolymere, Polyvinylacetate und/oder Acrylnitril-Butadien-Copolymere.

In einer weiteren bevorzugten Ausführungsform liegt mindestens ein Trennmittel (Antihaftmittel) oder Gleitmittel in der wärmeempfindlichen farbbildenden Schicht vor. Bei diesen Mitteln handelt es sich vorzugsweise um Fettsäure-Metallsalze, wie z. B. Zinkstearat oder Calciumstearat, oder auch Behenatsalze, synthetische Wachse, z. B. in Form von Fettsäureamiden, wie z. B. Stearinsäureamid und Behensäureamid, Fettsäurealkanolamide, wie z. B. Stearinsäuremethylolamid, Paraffinwachse verschiedener Schmelzpunkte, Esterwachse unterschiedlicher Molekulargewichte, Ethylenwachse, Propylenwachse unterschiedlicher Härten und/oder natürliche Wachse, wie z. B. Carnaubawachs oder Montanwachs.

Das Trennmittel liegt vorzugsweise in einer Menge von etwa 1 bis etwa 10 Gew.- %, besonders bevorzugt in einer Menge von etwa 3 bis etwa 6 Gew.-%, bezogen auf den gesamten Feststoffgehalt der wärmeempfindlichen Schicht, vor.

In einer weiteren bevorzugten Ausführungsform enthält die wärmeempfindliche farbbildende Schicht Pigmente. Der Einsatz dieser hat unter anderem den Vorteil, dass diese auf ihrer Oberfläche die im thermischen Druckprozess entstehende Chemikalien-Schmelze fixieren können. Auch kann über Pigmente die Oberflächenweiße und Opazität der wärmeempfindlichen farbbildenden Schicht und deren Bedruckbarkeit mit konventionellen Druckfarben gesteuert werden. Schließlich besitzen Pigmente eine "Extenderfunktion", beispielsweise für die relativ teuren farbgebenden Funktionschemikalien.

Besonders geeignete Pigmente sind anorganische Pigmente, sowohl synthetischer als auch natürlicher Herkunft, vorzugsweise Clays, gefällte oder natürliche Calciumcarbonate, Aluminiumoxide, Aluminiumhydroxide, Kieselsäuren, gefällte und pyrogene Kieselsäuren (z. B. Aerodisp®- Typen), Diathomeenerden, Magnesiumcarbonate, Talk, Kaolin, aber auch organische Pigmente, wie Hohlpigmente mit einer Styrol/Acrylat-Copolymer-Wand oder Harnstoff/Formaldehyd-Kondensationspolymere. Diese können alleine oder in beliebigen Mischungen verwendet werden.

Die Pigmente liegen vorzugsweise in einer Menge von etwa 20 bis etwa 50 Gew.-%, besonders bevorzugt in einer Menge von etwa 30 bis etwa 40 Gew.-%, bezogen auf den gesamten Feststoffgehalt der wärmeempfindlichen Schicht, vor.

Zum Steuern der Oberflächenweiße des erfindungsgemäßen wärmeempfindlichen Aufzeichnungsmaterials können optische Aufheller in die wärmeempfindliche farbbildende Schicht eingebaut werden. Bei diesen handelt es sich vorzugsweise um Stilbene.

Um bestimmte streichtechnische Eigenschaften zu verbessern, ist es im Einzelfall bevorzugt, zu den zwingenden Bestandteilen des erfindungsgemäßen wärmeempfindlichen Aufzeichnungsmaterials weitere Bestandteile, insbesondere Rheologie-Hilfsmittel, wie z. B. Verdicker und/oder Tenside, hinzuzufügen.

Das Flächenauftragsgewicht der (trockenen) wärmeempfindlichen Schicht beträgt vorzugsweise etwa 1 bis etwa 10 $g/m^2$, bevorzugt etwa 3 bis etwa 6 $g/m^2$.

In einer besonders bevorzugten Ausführungsform handelt es sich bei dem wärmeempfindlichen Aufzeichnungsmaterial um ein solches nach Anspruch 10, wobei als Farbbildner ein Farbstoff vom Fluoran-Typ eingesetzt wird und zusätzlich ein Sensibilisierungsmittel, ausgewählt aus der Gruppe bestehend aus Fettsäureamiden, aromatischen Sulfonen und/oder aromatischen Ethern, vorliegt. In dieser bevorzugten Ausführungsform ist es auch vorteilhaft, dass etwa 1,5 bis etwa 4 Gew.-Teile des phenolfreien Farbentwicklers nach Anspruch 1, bezogen auf 1 Gew.-Teil Farbbildner, vorliegen.

Die im Zusammenhang mit der Verbindung der Formel (I) aufgeführten bevorzugten Ausführungsformen gelten auch für das erfindungsgemäße wärmeempfindliche Aufzeichnungsmaterial.

Das erfindungsgemäße wärmeempfindliche Aufzeichnungsmaterial lässt sich mit bekannten Herstellungsverfahren gewinnen.

Es ist jedoch bevorzugt, das erfindungsgemäße Aufzeichnungsmaterial mit einem Verfahren zu gewinnen, bei dem auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-%, bevorzugt von etwa 30 bis etwa 50 Gew.-%, aufweist, und mit dem Curtain-Coating-Beschichtungsverfahren bei einer Betriebsgeschwindigkeit der Streichanlage von mindestens etwa 400 m/min aufgetragen und getrocknet wird.

Dieses Verfahren ist insbesondere unter wirtschaftlichen Gesichtspunkten vorteilhaft.

Wird der Wert des Feststoffgehaltes von etwa 20 Gew.-% unterschritten, dann verschlechtert sich die Wirtschaftlichkeit, da eine große Menge an Wasser aus dem Strich durch schonende Trocknung in kurzer Zeit entfernt werden muss, was sich nachteilig auf die Streichgeschwindigkeit auswirkt. Wird auf der anderen Seite der Wert von 75 Gew.-% überschritten, dann führt dies lediglich zu einem erhöhten technischen Aufwand, um die Stabilität des Streichfarben-Vorhangs während des Beschichtungsprozesses zu gewährleisten.

Beim Curtain-Coating-Beschichtungsverfahren (Vorhangbeschichtungsverfahren) wird ein frei fallender Vorhang einer Beschichtungsdispersion gebildet. Durch freien Fall wird die in Form eines dünnen Filmes (Vorhangs) vorliegende Beschichtungsdispersion auf ein Substrat "gegossen", um die Beschichtungsdispersion auf das Substrat aufzubringen. Die DE 10 196 052 T1 offenbart den Einsatz des Curtain-Coating-Beschichtungsverfahrens bei der Herstellung von Informationsaufzeichnungsmaterialien u.a. auch von wärmeempfindlichen Aufzeichnungsmaterialien, wobei mehrschichtige Aufzeichnungsschichten durch Aufbringen des aus mehreren Beschichtungsdispersionsfilmen bestehenden Vorhangs auf Substrate realisiert werden (Geschwindigkeit max. 200 m/min).

Die Einstellung der Betriebsgeschwindigkeit der Streichanlage auf mindestens etwa 400 m/min hat sowohl betriebswirtschaftliche als auch technische Vorteile. Bevorzugt beträgt die Betriebsgeschwindigkeit mindestens etwa 750 m/min, besonders bevorzugt mindestens etwa 1000 m/min und ganz besonders bevorzugt mindestens etwa 1500 m/min. Es war insbesondere überraschend, dass selbst bei letztgenannter Geschwindigkeit das erhaltene wärmeempfindliche Aufzeichnungsmaterial in keiner Weise beeinträchtigt ist und die Betriebsdurchführung selbst bei dieser hohen Geschwindigkeit optimal abläuft.

In einer bevorzugten Ausführungsform des erfindungsgemäßen Verfahrens weist die wässrige entlüftete Auftragssuspension eine Viskosität von etwa 150 bis etwa 800 mPas (Brookfield, 100 U/min, 20 °C) auf. Wird der Wert von etwa 150 mPas unterschritten bzw. der Wert von etwa 800 mPas überschritten, dann führt dies zu einer mangelhaften Lauffähigkeit der Streichmasse am Streichaggregat. Besonders bevorzugt beträgt die Viskosität der wässrigen entlüfteten Auftragssuspension etwa 200 bis etwa 500 mPas.

In einer bevorzugten Ausführungsform kann zur Optimierung des Verfahrens die Oberflächenspannung der wässrigen Auftragssuspension auf etwa 25 bis etwa 60 mN/m, bevorzugt auf etwa 35 bis etwa 50 mN/m (gemessen entsprechend der statischen Ringmethode nach Du Noüy, DIN 53914), eingestellt werden.

Die Ausbildung der wärmeempfindlichen farbbildenden Schicht kann on-line oder in einem separaten Streichvorgang off-line erfolgen. Dies gilt auch für eventuell nachfolgend aufgetragene Schichten oder Zwischenschichten.

Es ist vorteilhaft, wenn die getrocknete wärmeempfindliche farbbildende Schicht einer Glätt-Maßnahme unterzogen wird. Hierbei ist es vorteilhaft, die Bekk-Glätte, gemessen nach ISO 5627:1995-03, auf etwa 100 bis etwa 1000 sec., vorzugsweise auf etwa 250 bis etwa 600 sec., einzustellen.

Die Oberflächenrauigkeit (PPS) nach ISO 8791-4:2008-05 liegt vorzugsweise im Bereich von etwa 0,50 bis etwa 2,50 $\mu$m, besonders bevorzugt im Bereich von etwa 1,00 bis etwa 2,00 $\mu$m.

Die im Zusammenhang mit der Verbindung der Formel (I) aufgeführten bevorzugten Ausführungsformen gelten ebenfalls für das Verfahren zur Herstellung des erfindungsgemäßen wärmeempfindlichen Aufzeichnungsmaterials.

Die vorliegende Erfindung betrifft auch ein wärmeempfindliches Aufzeichnungsmaterial, welches mit dem vorstehend geschilderten Verfahren erhältlich ist.

Das vorstehend geschilderte Verfahren ist unter wirtschaftlichen Gesichtspunkten vorteilhaft und erlaubt eine hohe Verfahrensführung der Streichanlage sogar bei einer Geschwindigkeit von mehr als 1500 m/min, ohne dass es zu Beeinträchtigungen des Verfahrenserzeugnisses, das heißt des erfindungsgemäßen wärmeempfindlichen Aufzeichnungsmaterials, kommt. Die Verfahrensführung kann on-line und off-line erfolgen, was eine wünschenswerte Flexibilität zur Folge hat.

Das erfindungsgemäße wärmeempfindliche Aufzeichnungsmaterial ist phenolfrei und für POS(point-of-sale)-, Etiketten- und/oder Ticket-Anwendungen gut geeignet. Es eignet sich auch zur Herstellung von Parkscheinen, Fahrkarten, Eintrittskarten, Lotto- und Wettscheinen etc., welche im Thermodirektverfahren bedruckt werden können und eine hohe Beständigkeit der darauf aufgezeichneten Bilder auch bei längerfristiger Lagerung, selbst unter verschärften Klimabedingungen hinsichtlich Temperatur und Umgebungsfeuchte, benötigen.

Überraschenderweise hat es sich gezeigt, dass es möglich ist, mit den erfindungsgemäßen Farbentwicklern der Formel (I) wärmeempfindliche Aufzeichnungsmaterialien bereitzustellen, welche sich insbesondere dadurch auszeichnen, dass sie ihre Fähigkeit, hohe Bilddichten zu erzeugen, auch nach wochenlanger Lagerung der unbedruckten Materialien selbst bei hoher Umgebungsfeuchte und/oder Temperatur praktisch nicht einbüßen. Die erfindungsgemäßen wärmeempfindlichen Aufzeichnungsmaterialien zeigen daher eine überraschend lange Lagerfähigkeit.

Die Erfindung wird nachfolgend anhand nicht beschränkter Beispiele im Detail erläutert.

Beispiele:

Herstellung der erfindungsgemäßen Verbindungen der Formel (I).

Stufe A - Herstellung der Dinitro-Sulfonate (in Anlehnung an JP 2014 094 888 A)

Zu einer Lösung aus 60 mmol des entsprechenden Nitrophenols und 84 mmol Triethylamin in 20 mL THF wird eine Lösung aus 60 mmol des entsprechenden Nitrobenzolsulfonylchlorids in 15 mL THF bei Raumtemperatur unter Rühren tropfenweise zugegeben. Die Reaktionslösung wird zwei Stunden bei Raumtemperatur gerührt und anschließend mit 65 mL Wasser versetzt. Die Suspension wird drei Stunden bei Raumtemperatur gerührt und anschließend filtriert. Der Rückstand wird mit 10 mL Wasser (3 bis 6 mal) gewaschen. Die Dinitro-Sulfonate wurden ohne weitere Aufreinigung in der Stufe B eingesetzt.

Stufe B - Reduktion der Nitro-Gruppen zu primären Aminen

Zu einer Lösung aus 0,060 mol des Produktes aus Stufe A in 200 mL Ethylacetat werden unter Rühren 0,420 mol $SnCl_2 \cdot 2H_2O$ (portionsweise) bei Raumtemperatur zugegeben. Die Reaktionslösung wird refluxiert. Der Reaktionsverlauf wird mittels Dünnschichtchromatographie verfolgt (Eluenten: Cyclohexan/Ethylacetat 1:1). Nach Beenden der Reaktion (etwa 2 bis 3 h) wird mit 200 mL einer 50%igen wässrigen Kaliumcarbonat-Lösung versetzt und 30 min bei Raumtemperatur gerührt. Die Phasen werden voneinander getrennt. Die wässrige Phase wird mit 100 mL Ethylacetat extrahiert. Die vereinigten organischen Phasen werden über Magnesiumsulfat getrocknet. Das Lösungsmittel wird im Vakuum entfernt. Die Diaminosulfonate werden ohne weitere Aufreinigung in der Stufe C eingesetzt.

Stufe C - Herstellung der Bis-Harnstoff-Verbindungen

Zu einer Lösung aus 7,6 mmol des Produktes aus Stufe B in 40 mL Ethylacetat wird eine Lösung aus 15,2 mmol des entsprechenden Isocyanats in 25 mL Ethylacetat bei Raumtemperatur unter Rühren tropfenweise zugegeben. Das Reaktionsgemisch wird refluxiert. Das Reaktionsgemisch wird refluxiert und der Reaktionsverlauf mittels HPLC verfolgt. Nach Beenden der Reaktion wird der ausgefallene Bis-Harnstoff abfiltriert, mit 10 mL Ethylacetat (3mal) gewaschen und im Vakuum getrocknet. Gelegentlich erfolgt eine weitere Aufreinigung durch Umkristallisieren aus Ethylacetat oder Dichlormethan. In manchen Fällen wird die Reaktionslösung im Vakuum eingeengt und die Kristallisation durch Zugabe weniger Tropfen n-Hexan ausgelöst.

Die Ausgangsverbindungen sind kommerziell erhältlich.

Tabelle 2 fasst die hier erstmalig hergestellten Verbindungen der Formel (I) zusammen.

Tabelle 2: Zusammenstellung erstmalig hergestellter Verbindungen der Formel (I)

| | Anordnung von $Ar^1$-NH-CO-NH- und $Ar^2$-NH-CO-NH- an der - $C_6H_4$-$SO_2$-O-$C_6H_4$-Gruppe | $Ar^1$ | $Ar^2$ |
|---|---|---|---|
| **I** | 2,3' | $C_6H_5$ | $C_6H_5$ |
| **II** | 2,4' | $C_6H_5$ | $C_6H_5$ |
| **III** | 3,2' | $C_6H_5$ | $C_6H_5$ |
| **IV** | 3,3' | $C_6H_5$ | $C_6H_5$ |
| **V** | 3,4' | $C_6H_5$ | $C_6H_5$ |
| **VI** | 4,2' | $C_6H_5$ | $C_6H_5$ |
| **VII** | 4,3' | $C_6H_5$ | $C_6H_5$ |
| **VIII** | 4,4' | $C_6H_5$ | $C_6H_5$ |
| **IX** | 4,3' | 2-$CH_3$-$C_6H_4$ | 2-$CH_3$-$C_6H_4$ |
| **X** | 4,3' | 3-$CH_3$-$C_6H_4$ | 3-$CH_3$-$C_6H_4$ |
| **XI** | 4,3' | 4-$CH_3$-$C_6H_4$ | 4-$CH_3$-$C_6H_4$ |
| **XII** | 4,3' | 2,4,6-Tri$CH_3$-$C_6H_2$ | 2,4,6-Tri$CH_3$-$C_6H_2$ |
| **XIII** | 4,3' | 1-Naph | 1-Naph |
| **XIV** | 4,3' | 4-$CH_3O$-$C_6H_4$ | 4-$CH_3O$-$C_6H_4$ |

(fortgesetzt)

| | Anordnung von $Ar^1$-NH-CO-NH- und $Ar^2$-NH-CO-NH- an der - $C_6H_4$-$SO_2$-O-$C_6H_4$-Gruppe | $Ar^1$ | $Ar^2$ |
|---|---|---|---|
| **XV** | 4,3' | 4-Cl-$C_6H_4$ | 4-Cl-$C_6H_4$ |
| **XVI** | 4,3' | 4-F-$C_6H_4$ | 4-F-$C_6H_4$ |
| **XVII** | 4,3' | 4-$CH_3CO$-$C_6H_4$ | 4-$CH_3CO$-$C_6H_4$ |
| **XVIII** | 4,3' | 4-($CO_2C_2H_5$)-$C_6H_4$ | 4-($CO_2C_2H_5$)-$C_6H_4$ |
| **XIX** | 4,3' | 4-CN-$C_6H_4$ | 4-CN-$C_6H_4$ |
| **XX** | 4,3' | 4-$NO_2$-$C_6H_4$ | 4-$NO_2$-$C_6H_4$ |
| **XXI** | 4,3' | 4-$CH_3O$-$C_6H_4$ | 4-$NO_2$-$C_6H_4$ |
| **XXII** | 4,3' | 4-$NO_2$-$C_6H_4$ | 4-$CH_3O$-$C_6H_4$ |

Analytische Daten:

**I,** $C_{26}H_{22}N_4O_5S$, M = 502.5, 3'-(3'-Phenylureido)phenyl 2-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.88 (1H, s), 8.82 (1H, s), 8.63 (1H, s), 8.43 (1H, s), 8.17-8.16 (1H, m), 7.75-7.70 (2H, m), 7.53-7.51 (2H, m), 7.46-7.43 (3H, m), 7.32-7.26 (5H, m), 7.23-7.20 (2H, m), 7.03-6.97 (2H, m), 6.55 (1H, ddd, *J* = 8.0, 2.4, 1.0 Hz).

$^{13}$C-NMR (126 MHz, DMSO-$d_6$): δ (ppm) = 152.13 (NHCONH), 151.80 (NHCONH), 149.06, 141.21, 139.25, 139.25, 137.88, 135.43, 129.94, 129.87, 128.71, 128.66, 124.57, 122.82, 122.69, 122.31, 122.00, 118.60, 118.34, 116.89, 114.27, 111.37.

**II,** $C_{26}H_{22}N_4O_5S$, M = 502.5, 4'-(3'-Phenylureido)phenyl 2-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.88 (1H, s), 8.73 (1H, s), 8.63 (1H, s), 8.42 (1H, s), 8.13 (1H, dd, *J* = 8.4, 1.0 Hz), 7.76-7.72 (1H, m), 7.66 (1H, dd, *J* = 8.1, 1.5 Hz), 7.52-7.50 (2H, m), 7.43-7.40 (4H, m), 7.32-7.29 (2H, m), 7.28-7.25 (2H, m), 7.22-7.19 (1H, m), 7.02-6.99 (1H, m), 6.98-6.93 (3H, m).

$^{13}$C-NMR (126 MHz, DMSO-$d_6$): δ (ppm) = 152.25 (NHCONH), 151.88 (NHCONH), 142.82, 139.37, 139.23, 138.99, 137.83, 135.39, 130.04, 128.73, 128.64, 124.83, 122.92, 122.80, 122.32, 122.14, 121.89, 118.96, 118.58, 118.25.

**III,** $C_{26}H_{22}N_4O_5S$, M = 502.5, 2'-(3'-Phenylureido)phenyl 3-(3-phenylureido)benzolsulfonat

MS (ESI): m/z (%) = 503.2 (100) $[M+H]^+$.

$^1$H-NMR (500 MHz, DMSO-$d_6$): δ (ppm) = 9.05 (1H, s), 8.98 (1H, s), 8.60 (1H, s), 8.27-8.27 (1H, m), 8.19 (1H, s), 8.03 (1H, dd, J = 8.3, 1.3 Hz), 7.54-7.52 (1H, m), 7.43-7.37 (6H, m), 7.30-7.27 (2H, m), 7.24-7.19 (3H, m), 7.13 (1H, dd, J = 8.2, 1.2 Hz), 7.01-6.97 (2H, m), 6.96-6.93 (1H, m).

$^{13}$C-NMR (126 MHz, DMSO-$d_6$): δ (ppm) = 152.08 (NHCONH), 151.53 (NHCONH), 140.66, 139.32, 139.10, 138.10, 134.47, 132.13, 129.67, 128.63, 128.58, 127.45, 123.73, 122.11, 122.07, 122.03, 121.81, 121.13, 121.03, 118.43, 118.05, 116.91.

**IV,** $C_{26}H_{22}N_4O_5S$, M = 502.5, 3'-(3'-Phenylureido)phenyl 3-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.13 (1H, s), 8.86 (1H, s), 8.73 (1H, s), 8.62 (1H, s), 8.23-8.22 (1H, m), 7.72 (1H, ddd, *J* = 8.2, 2.2, 1.0 Hz), 7.58-7.55 (1H, m), 7.48-7.42 (6H, m), 7.31-7.25 (6H, m), 7.00 (1H, tt, *J* = 7.4, 1.1 Hz), 6.98 (1H, tt, *J* = 7.4, 1.1 Hz), 6.62 (1H, ddd, *J* = 7.6, 2.3, 1.6 Hz).

$^{13}$C-NMR (126 MHz, DMSO-$d_6$): δ (ppm) = 152.30 (NHCONH), 152.18 (NHCONH), 149.34, 141.17, 140.91, 139.27, 139.11, 135.07, 130.09, 129.86, 128.68, 128.67, 123.76, 122.22, 122.01, 120.87, 118.59, 118.37, 116.67,

116.61, 114.58, 111.38.

**V,** $C_{26}H_{22}N_4O_5S$, M = 502.5, 4'-(3'-Phenylureido)phenyl 3-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.13 (1H, s), 8.76 (1H, s), 8.74 (1H, s), 8.63 (1H, s), 8.19-8.19 (1H, m), 7.72 (1H, ddd, *J* = 8.2, 2.2, 1.0 Hz), 7.58-7.55 (1H, m), 7.48-7.42 (6H, m), 7.41 (1H, ddd, *J* = 7.8, 1.8, 1.0 Hz), 7.31-7.25 (4H, m), 7.01-6.95 (4H, m).

$^{13}$C-NMR (126 MHz, DMSO-$d_6$): δ (ppm) = 152.34 (NHCONH), 152.33 (NHCONH), 143.22, 140.88, 139.40, 139.12, 138.78, 134.97, 130.06, 128.69, 128.67, 123.76, 122.31, 122.24, 121.92, 120.99, 119.04, 118.62, 118.30, 116.74.

**VI,** $C_{26}H_{22}N_4O_5S$, M = 502.5, 2'-(3'-Phenylureido)phenyl 4-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.15 (1H, s), 9.10 (1H, s), 8.71 (1H, s), 8.13 (1H, s), 8.05 (1H, dd, *J* = 8.3, 1.5 Hz), 7.75-7.74 (2H, m), 7.60-7.58 (2H, m), 7.44-7.42 (2H, m), 7.42-7.40 (2H, m), 7.31-7.21 (5H, m), 7.14 (1H, dd, *J* = 8.2, 1.4 Hz), 7.02-6.97 (2H, m), 6.95-6.92 (1H, m).

$^{13}$C-NMR (126 MHz, DMSO-$d_6$): δ (ppm) = 151.71 (NHCONH), 151.62 (NHCONH), 145.83, 139.25, 138.91, 138.07, 132.15, 129.83, 128.68, 128.63, 127.30, 125.32, 122.32, 122.18, 122.01, 121.90, 120.94, 118.54, 118.29, 117.19.

**VII,** $C_{26}H_{22}N_4O_5S$, M = 502.5, 3'-(3'-Phenylureido)phenyl 4-(3-phenylureido)benzolsulfonat

MS (ESI): m/z (%) = 503.1 (100) $[M+H]^+$.

$^1$H-NMR (500 MHz, DMSO-$d_6$): δ (ppm) = 9.28 (1H, s), 8.84 (1H, s), 8.81 (1H, s), 8.62 (1H, s), 7.79-7.77 (2H, m), 7.72-7.71 (2H, m), 7.48-7.46 (2H, m), 7.45-7.43 (2H, m), 7.38-7.38 (1H, m), 7.32-7.24 (6H, m), 7.03-7.00 (1H, m), 6.98-6.95 (1H, m), 6.60-6.59 (1H, m).

$^{13}$C-NMR (126 MHz, DMSO-$d_6$): δ (ppm) = 152.16 (NHCONH), 151.93 (NHCONH), 149.43, 145.61, 141.08, 139.27, 138.94, 129.76, 129.56, 128.71, 128.64, 125.98, 122.36, 121.96, 118.56, 118.35, 117.60, 116.51, 114.74, 111.47.

**VIII,** $C_{26}H_{22}N_4O_5S$, M = 502.5, 4'-(3'-Phenylureido)phenyl 4-(3-phenylureido)benzolsulfonat

MS (ESI): m/z (%) = 503.2 (100) $[M+H]^+$.

$^1$H-NMR (500 MHz, DMSO-$d_6$): δ (ppm) = 9.28 (1H, s), 8.82 (1H, s), 8.74 (1H, s), 8.63 (1H, s), 7.75-7.73 (2H, m), 7.71-7.70 (2H, m), 7.48-7.47 (2H, m), 7.44-7.43 (4H, m), 7.32-7.29 (2H, m), 7.29-7.25 (2H, m), 7.03-7.00 (1H, m), 6.98-6.96 (1H, m), 6.96-6.92 (2H, m).

$^{13}$C-NMR (126 MHz, DMSO-$d_6$): δ (ppm) = 152.32 (NHCONH), 151.94 (NHCONH), 145.56, 143.28, 139.40, 138.95, 138.63, 129.62, 128.72, 128.64, 125.89, 122.39, 122.37, 121.87, 118.96, 118.57, 118.26, 117.58.

**IX,** $C_{28}H_{26}N_4O_5S$, M = 530.6, 3'-(3'-(2'-Tolyl)ureido)phenyl 4-(3-(2-tolyl)ureido)benzolsulfonat

MS (ESI): m/z (%) = 531.1 (100) $[M+H]^+$.

$^1$H-NMR (500 MHz, DMSO-$d_6$): δ (ppm) = 9.58 (1H, s), 9.17 (1H, s), 8.09 (1H, s), 7.90 (1H, s), 7.79-7.76 (4H, m), 7.73-7.70 (2H, m), 7.40-7.39 (1H, m), 7.31-7.28 (1H, m), 7.27-7.24 (1H, m), 7.20-7.12 (4H, m), 7.01-6.98 (1H, m), 6.97-6.93 (1H, m), 6.59 (1H, ddd, *J* = 7.7, 2.4, 1.4 Hz), 2.25 (3H, s), 2.23 (3H, s).

$^{13}$C-NMR (126 MHz, DMSO-$d_6$): δ (ppm) = 152.31 (NHCONH), 152.11 (NHCONH), 149.46, 145.75, 141.25, 136.96, 136.61, 130.15, 130.06, 129.81, 129.61, 128.35, 127.88, 126.07, 126.03, 125.84, 123.37, 122.91, 121.70, 121.36, 117.44, 116.29, 114.63, 111.29, 17.68 ($CH_3$), 17.68 ($CH_3$).

**X,** $C_{28}H_{26}N_4O_5S$, M = 530.6, 3'-(3'-(3'-Tolyl)ureido)phenyl 4-(3-(3-tolyl)ureido)benzolsulfonat

MS (ESI): m/z (%) = 531.1 (100) $[M+H]^+$.

$^1$H-NMR (500 MHz, DMSO-$d_6$): δ (ppm) = 9.26 (1H, s), 8.83 (1H, s), 8.73 (1H, s), 8.54 (1H, s), 7.78-7.76 (2H, m), 7.72-7.70 (2H, m), 7.40-7.39 (1H, m), 7.31-7.31 (1H, m), 7.29-7.21 (5H, m), 7.19-7.13 (2H, m), 6.84-6.82 (1H, m), 6.80-6.78 (1H, m), 6.59 (1H, ddd, *J* = 7.6, 2.4, 1.5 Hz), 2.29 (3H, s), 2.27 (3H, s).

$^{13}$C-NMR (126 MHz, DMSO-$d_6$): δ (ppm) = 152.14 (NHCONH), 151.90 (NHCONH), 149.43, 145.63, 141.12, 139.19, 138.86, 137.94, 137.86, 129.75, 129.55, 128.55, 128.49, 125.95, 123.11, 122.72, 119.08, 118.85, 117.58, 116.47, 115.74, 115.53, 114.69, 111.44, 21.07 ($CH_3$), 21.07 ($CH_3$).

**XI,** $C_{28}H_{26}N_4O_5S$, M = 530.6, 3'-(3'-(4'-Tolyl)ureido)phenyl 4-(3-(4-tolyl)ureido)benzolsulfonat

MS (ESI): m/z (%) = 531.1 (100) $[M+H]^+$.

$^{1}$H-NMR (500 MHz, DMSO-$d_6$): δ (ppm) = 9.23 (1H, s), 8.79 (1H, s), 8.71 (1H, s), 8.50 (1H, s), 7.77-7.75 (2H, m), 7.71-7.70 (2H, m), 7.37-7.35 (3H, m), 7.33-7.31 (2H, m), 7.26-7.23 (2H, m), 7.11-7.10 (2H, m), 7.07-7.05 (2H, m), 6.60-6.57 (1H, m), 2.25 (3H, s), 2.21 (3H, s).

$^{13}$C-NMR (126 MHz, DMSO-$d_6$): δ (ppm) = 152.18 (NHCONH), 151.94 (NHCONH), 149.44, 145.72, 141.18, 136.69, 136.38, 131.29, 130.82, 129.73, 129.56, 129.11, 129.05, 125.85, 118.65, 118.44, 117.53, 116.43, 114.66, 111.41, 20.24 ($CH_3$), 20.19 ($CH_3$).

**XII,** $C_{32}H_{34}N_4O_5S$, M = 586.7, 3'-(3'-(2',4',6'-Trimethylphenyl)ureido)phenyl 4-(3-(2,4,6-trimethylphenyl)ureido)benzolsulfonat

MS (ESI): m/z (%) = 587.2 (100) $[M+H]^+$.

$^{1}$H-NMR (500 MHz, DMSO-$d_6$): δ (ppm) = 9.34 (1H, s), 8.86 (1H, s), 7.82 (1H, s), 7.73-7.71 (2H, m), 7.69-7.67 (2H, m), 7.61 (1H, s), 7.35-7.35 (1H, m), 7.29-7.28 (1H, m), 7.23-7.20 (1H, m), 6.90-6.87 (4H, m), 6.54-6.53 (1H, m), 2.23 (3H, s), 2.23 (3H, s), 2.17 (6H, s), 2.14 (6H, s).

$^{13}$C-NMR (126 MHz, DMSO-$d_6$): δ (ppm) = 152.90 (NHCONH), 152.59 (NHCONH), 149.42, 146.17, 141.73, 135.24, 135.22, 135.20, 134.94, 132.29, 131.99, 129.62, 129.49, 128.26, 128.19, 125.50, 117.25, 116.19, 114.21, 111.23, 20.37 ($CH_3$), 17.98 ($CH_3$).

**XIII,** $C_{34}H_{26}N_4O_5S$, M = 602.7,3'-(3'-(1'-Naphthyl)ureido)phenyl 4-(3-(1-naphthyl)ureido)benzolsulfonat

MS (ESI): m/z (%) = 603.2 (100) $[M+H]^+$.

$^{1}$H-NMR (500 MHz, DMSO-$d_6$): δ (ppm) = 9.64 (1H, s), 9.23 (1H, s), 8.91 (1H, s), 8.75 (1H, s), 8.12-8.09 (2H, m), 7.99-7.96 (2H, m), 7.95-7.94 (1H, m), 7.92-7.91 (1H, m), 7.83-7.81 (2H, m), 7.79-7.77 (2H, m), 7.70-7.68 (1H, m), 7.64-7.62 (1H, m), 7.61-7.45 (7H, m), 7.35 (1H, ddd, *J* = 8.2, 2.0, 1.1 Hz), 7.31-7.28 (1H, m), 6.63 (1H, ddd, *J* = 8.0, 2.4, 1.0 Hz).

$^{13}$C-NMR (126 MHz, DMSO-$d_6$): δ (ppm) = 152.63 (NHCONH), 152.48 (NHCONH), 149.51, 145.70, 141.18, 133.90, 133.64, 133.62, 133.58, 129.90, 129.69, 128.32, 128.30, 126.32, 126.08, 126.00, 125.90, 125.80, 125.78, 125.73, 125.70, 125.65, 123.70, 123.19, 121.33, 121.25, 118.34, 117.81, 117.62, 116.47, 114.82, 111.44.

**XIV,** $C_{28}H_{26}N_4O_7S$, M = 562.6, 3'-(3'-(4'-Methoxyphenyl)ureido)phenyl 4-(3-(4-methoxyphenyl)ureido)benzolsulfonat

MS (ESI): m/z (%) = 563.2 (100) $[M+H]^+$.

$^{1}$H-NMR (500 MHz, DMSO-$d_6$): δ (ppm) = 9.21 (1H, s), 8.76 (1H, s), 8.63 (1H, s), 8.42 (1H, s), 7.77-7.74 (2H, m), 7.71-7.69 (2H, m), 7.39-7.32 (5H, m), 7.28-7.22 (2H, m), 6.90-6.87 (2H, m), 6.87-6.84 (2H, m), 6.58 (1H, ddd, *J* = 7.4, 2.3, 1.7 Hz), 3.73 (3H, s), 3.69 (3H, s).

$^{13}$C-NMR (126 MHz, DMSO-$d_6$): δ (ppm) = 154.89, 154.61, 152.34 (NHCONH), 152.09 (NHCONH), 149.44, 145.81, 141.28, 132.28, 131.92, 129.71, 129.55, 125.74, 120.45, 120.23, 117.48, 116.39, 114.55, 113.99, 113.92, 111.37, 55.14 ($OCH_3$), 55.07 ($OCH_3$).

**XV,** $C_{26}H_{20}Cl_2N_4O_5S$, M = 571.4, 3'-(3'-(4'-Chlorphenyl)ureido)phenyl 4-(3-(4-chlorphenyl)ureido)benzolsulfonat

MS (ESI): m/z (%) = 571.0 (100) $[M+H]^+$.

$^{1}$H-NMR (500 MHz, DMSO-$d_6$): δ (ppm) = 9.31 (1H, s), 8.95 (1H, s), 8.87 (1H, s), 8.76 (1H, s), 7.78-7.76 (2H, m), 7.72-7.69 (2H, m), 7.51-7.48 (2H, m), 7.48-7.45 (2H, m), 7.37-7.36 (1H, m), 7.36-7.33 (2H, m), 7.31-7.28 (2H, m), 7.28-7.25 (2H, m), 6.61 (1H, ddd, *J* = 6.0, 3.1, 2.4 Hz).

$^{13}$C-NMR (126 MHz, DMSO-$d_6$): δ (ppm) = 152.05 (NHCONH), 151.86 (NHCONH), 149.41, 145.45, 140.88, 138.29, 137.96, 129.77, 129.56, 128.57, 128.47, 126.13, 126.00, 125.55, 120.09, 119.84, 117.74, 116.62, 114.94, 111.58.

**XVI,** $C_{26}H_{20}F_2N_4O_5S$, M = 538.5, 3'-(3'-(4'-Fluorphenyl)ureido)phenyl 4-(3-(4-fluorphenyl)ureido)benzolsulfonat

MS (ESI): m/z (%) = 539.2 (100) $[M+H]^+$.

$^{1}$H-NMR (500 MHz, DMSO-$d_6$): δ (ppm) = 9.28 (1H, s), 8.85 (1H, s), 8.84 (1H, s), 8.65 (1H, s), 7.78-7.75 (2H, m), 7.72-7.69 (2H, m), 7.50-7.42 (4H, m), 7.37-7.36 (1H, m), 7.29-7.23 (2H, m), 7.16-7.06 (4H, m), 6.60 (1H, ddd, *J* = 7.5, 2.3, 1.6 Hz).

$^{13}$C-NMR (126 MHz, DMSO-$d_6$): δ (ppm) = 158.49 (d, *J* = 29.7 Hz), 156.59 (d, *J* = 29.3 Hz), 152.26 (NHCONH),

152.05 (NHCONH), 149.43, 145.60, 141.06, 135.60 (d, *J* = 2.3 Hz), 135.26 (d, *J* = 2.3 Hz), 129.75, 129.56, 126.01, 120.45 (d, *J* = 7.8 Hz), 120.17 (d, *J* = 7.7 Hz), 117.65, 116.56, 115.27 (d, *J* = 13.4 Hz), 115.10 (d, *J* = 13.4 Hz), 114.78, 111.52.

**XVII,** $C_{30}H_{26}N_4O_7S$, M = 586.6, 3'-(3'-(4'-Acetylphenyl)ureido)phenyl 4-(3-(4-acetylphenyl)ureido)benzolsulfonat

MS (ESI): m/z (%) = 587.1 (100) $[M+H]^+$.

$^1$H-NMR (500 MHz, DMSO-$d_6$): δ (ppm) = 9.41 (1H, s), 9.24 (1H, s), 9.06 (1H, s), 8.98 (1H, s), 7.92-7.91 (2H, m), 7.89-7.87 (2H, m), 7.80-7.78 (2H, m), 7.74-7.73 (2H, m), 7.60-7.59 (2H, m), 7.57-7.56 (2H, m), 7.38-7.38 (1H, m), 7.28-7.27 (2H, m), 6.65-6.63 (1H, m), 2.52 (3H, s), 2.46 (3H, s).

$^{13}$C-NMR (126 MHz, DMSO-$d_6$): δ (ppm) = 196.23 ($COCH_3$), 196.14 ($COCH_3$), 151.83 (NHCONH), 151.69 (NHCONH), 149.43, 145.29, 143.94, 143.58, 140.69, 130.95, 130.61, 129.89, 129.66, 129.54, 129.51, 126.34, 117.94, 117.51, 117.27, 116.81, 115.27, 111.75, 26.26 ($CH_3$), 26.17 ($CH_3$).

**XVIII,** $C_{32}H_{30}N_4O_9S$, M = 646.7, Ethyl 4-(3-(4-((3-(3-(4-(ethoxycarbonyl)phenyl)ureido)phenoxy)sulfonyl)phenyl)ureido)benzoat

MS (ESI): m/z (%) = 647.2 (100) $[M+H]^+$.

$^1$H-NMR (500 MHz, DMSO-$d_6$): δ (ppm) = 9.38 (1H, s), 9.21 (1H, s), 9.03 (1H, s), 8.95 (1H, s), 7.91-7.89 (2H, m), 7.88-7.86 (2H, m), 7.80-7.77 (2H, m), 7.74-7.72 (2H, m), 7.60-7.58 (2H, m), 7.58-7.55 (2H, m), 7.38-7.37 (1H, m), 7.28-7.27 (2H, m), 6.66-6.63 (1H, m), 4.29 (2H, q, *J* = 7.1 Hz), 4.26 (2H, q, *J* = 7.1 Hz), 1.31 (3H, q, *J* = 7.1 Hz), 1.29 (3H, q, *J* = 7.1 Hz).

$^{13}$C-NMR (126 MHz, DMSO-$d_6$): δ (ppm) = 165.27 (COO), 165.26 (COO), 151.79 (NHCONH), 151.65 (NHCONH), 149.40, 145.26, 143.87, 143.52, 140.65, 130.21, 130.20, 129.82, 129.59, 126.35, 123.34, 122.96, 117.90, 117.61, 117.36, 116.75, 115.22, 111.71, 60.21, 60.13 ($CH_2$), 14.11 ($CH_3$).

**XIX,** $C_{28}H_{20}N_6O_5S$, M = 552.6, 3'-(3'-(4'-Cyanophenyl)ureido)phenyl 4-(3-(4-cyanophenyl)ureido)benzolsulfonat

MS (ESI): m/z (%) = 553.1 (100) $[M+H]^+$.

$^1$H-NMR (500 MHz, DMSO-$d_6$): δ (ppm) = 9.45 (1H, s), 9.32 (1H, s), 9.14 (1H, s), 9.02 (1H, s), 7.80-7.77 (2H, m), 7.76-7.71 (4H, m), 7.70-7.67 (2H, m), 7.65-7.63 (2H, m), 7.62-7.60 (2H, m), 7.36-7.36 (1H, m), 7.28-7.27 (2H, m), 6.66-6.63 (1H, m).

$^{13}$C-NMR (126 MHz, DMSO-$d_6$): δ (ppm) = 151.74 (NHCONH), 151.62 (NHCONH), 149.37, 145.10, 143.79, 143.45, 140.50, 133.20, 133.10, 129.88, 129.61, 126.50, 119.05, 119.03, 118.36, 118.13, 118.01, 116.88, 115.37, 111.80, 103.95, 103.46.

**XX,** $C_{26}H_{20}N_6O_9S$, M = 592.5, 3'-(3'-(4'-Nitrophenyl)ureido)phenyl 4-(3-(4-nitrophenyl)ureido)benzolsulfonat

MS (ESI): m/z (%) = 593.1 (100) $[M+H]^+$.

$^1$H-NMR (500 MHz, DMSO-$d_6$): δ (ppm) = 9.52 (1H, s), 9.48 (1H, s), 9.36 (1H, s), 9.06 (1H, s), 8.20-8.17 (2H, m), 8.15-8.12 (2H, m), 7.81-7.78 (2H, m), 7.75-7.73 (2H, m), 7.70-7.64 (4H, m), 7.37-7.36 (1H, m), 7.31-7.27 (2H, m), 6.69-6.66 (1H, m).

$^{13}$C-NMR (126 MHz, DMSO-$d_6$): δ (ppm) = 151.60 (NHCONH), 151.51 (NHCONH), 149.38, 145.93, 145.54, 145.00, 141.46, 141.12, 140.36, 129.88, 129.62, 126.64, 124.93, 124.90, 118.13, 117.79, 117.53, 116.97, 115.57, 111.91.

**XXI,** $C_{27}H_{23}N_5O_8S$, M = 577.6, 3'-(3'-(4'-Nitrophenyl)ureido)phenyl 4-(3-(4-methoxyphenyl)ureido)benzolsulfonat

MS (ESI): m/z (%) = 578.1 (100) $[M+H]^+$.

$^1$H-NMR (500 MHz, DMSO-$d_6$): δ (ppm) = 9.37 (1H, s), 9.20 (1H, s), 9.07 (1H, s), 8.62 (1H, s), 8.18-8.15 (2H, m), 7.76-7.74 (2H, m), 7.71-7.66 (4H, m), 7.37-7.34 (3H, m), 7.31-7.27 (2H, m), 6.89-6.86 (2H, m), 6.67-6.65 (1H, m), 3.72 (3H, s).

$^{13}$C-NMR (126 MHz, DMSO-$d_6$): δ (ppm) = 154.89, 152.08 (NHCONH), 151.64 (NHCONH), 149.42, 145.94, 145.87, 141.17, 140.36, 131.88, 129.87, 129.57, 125.63, 124.94, 120.44, 117.59, 117.51, 116.95, 115.56, 113.97, 111.94, 55.13 ($OCH_3$).

**XXII,** $C_{27}H_{23}N_5O_8S$, M = 577.6, 3'-(3'-(4'-Methoxyphenyl)ureido)phenyl 4-(3-(4-nitrophenyl)ureido)benzolsulfonat

MS (ESI): m/z (%) = 578.1 (100) $[M+H]^+$.

$^1$H-NMR (500 MHz, DMSO-$d_6$): δ (ppm) = 9.52 (2H, s), 8.75 (1H, s), 8.42 (1H, s), 8.22-8.19 (2H, m), 7.81-7.79 (2H, m), 7.75-7.73 (2H, m), 7.73-7.70 (2H, m), 7.37-7.36 (1H, m), 7.35-7.31 (2H, m), 7.26-7.22 (2H, m), 6.86-6.82 (2H, m), 6.60-6.56 (1H, m), 3.68 (3H, s).

$^{13}$C-NMR (126 MHz, DMSO-$d_6$): δ (ppm) = 154.60, 152.33 (NHCONH), 151.55 (NHCONH), 149.41, 145.61, 144.94, 141.47, 141.30, 132.28, 129.73, 129.60, 126.77, 124.98, 120.20, 118.11, 117.86, 116.43, 114.53, 113.91, 111.34, 55.07 ($OCH_3$).

Als Vergleichsfarbentwickler wurden nicht-phenolische Farbentwickler des Standes der Technik herangezogen, nämlich *N*-(2-(3-Phenylureido)phenyl) benzolsulfonamid (**NKK 1304,** Nippon Soda) sowie ein Sulfonylharnstoff, Pergafast 201® (**PF 201,** BASF).

Der Auftrag einer wässrigen Auftragssuspension zur Ausbildung der wärmeempfindlichen farbbildenden Schicht eines wärmeempfindlichen Aufzeichnungspapiers erfolgte im Labormaßstab mittels einer Stabrakel auf eine Seite eines synthetischen Basispapieres (Yupo® FP680) von 63 g/m$^2$. Nach Trocknung wurde ein thermisches Aufzeichnungsblatt erhalten. Die Auftragsmenge der wärmeempfindlichen farbbildenden Schicht lag zwischen 3,8 und 4,2 g/m$^2$.

Anhand der vorstehend gemachten Angaben wurde ein wärmeempfindliches Aufzeichnungsmaterial bzw. Thermopapier hergestellt, wobei die folgenden Rezepturen wässriger Auftragssuspensionen zur Ausbildung eines Verbundgebildes auf einem Trägersubstrat herangezogen und anschließend in üblicher Weise die weiteren Schichten, insbesondere eine Schutzschicht, ausgebildet wurden, worauf hier nicht gesondert eingegangen werden soll.

Herstellen der Dispersionen (jeweils für 1 Gew.-Teil) für die Auftragssuspensionen

Die wässrige **Dispersion A** (Farbbildnerdispersion) wird durch Mahlen von 20 Gew.-Teilen 3-*N*-n-Dibutylamin-6-methyl-7-anilinofluoran (ODB-2) mit 33 Gew.-Teilen einer 15%igen wässrigen Lösung von Ghosenex™ L-3266 (sulfonierter Polyvinylalkohol, Nippon Ghosei) in einer Perlen-Mühle hergestellt.

Die wässrige **Dispersion B** (Farbentwicklerdispersion) wird durch Mahlen von 40 Gew.-Teilen des Farbentwicklers zusammen mit 66 Gew.-Teilen einer 15%igen wässrigen Lösung von Ghosenex™ L-3266 in einer Perlen-Mühle hergestellt.

Die wässrige **Dispersion C** (Sensibilisierungsdispersion) wird durch Mahlen von 40 Gew.-Teilen 1,2-Diphenoxyethan mit 33 Gew.-Teilen einer 15%igen wässrigen Lösung von Ghosenex™ L-3266 in einer Perlen-Mühle hergestellt.

Alle durch Mahlen erzeugten Dispersionen haben eine mittlere Korngröße $D_{(4,3)}$ von 0,80-1,20 μm. Die Messung der Korngrößenverteilung der Dispersionen erfolgte durch Laserbeugung mit einem Coulter LS230-Gerät der Fa. Beckman Coulter.

Die **Dispersion D** (Gleitmitteldispersion) ist eine 20%ige Zinkstearat-Dispersion, bestehend aus 9 Gew.-Teilen Zn-Stearat, 1 Gew.-Teil Ghosenex™ L-3266 und 40 Gew.-Teilen Wasser.

Pigment **P** ist eine 72%ige Streichkaolin-Suspension (Lustra® S, BASF).

Der **Binder** besteht aus einer 10%igen wässrigen Polyvinylalkohollösung (Poval 28-99, Kuraray Europe).

Die wärmeempfindliche Auftragssuspension wird durch Mischen unter Rühren von 1 Teil **A,** 1 Teil **B,** 1 Teil **C,** 56 Teilen **D,** 146 Teilen Pigment **P** und 138 Teilen **Binder-**Lösung (alles Gew.-Teile) unter Berücksichtigung der Eintragsreihenfolge **B, D, C, P, A, Binder** hergestellt und mit Wasser auf einen Feststoffgehalt von etwa 25% gebracht.

Die so erhaltenen wärmeempfindlichen Beschichtungssuspensionen wurden herangezogen, um Verbundgebilde aus Papierträger und Thermoreaktionsschicht herzustellen.

Die thermischen Aufzeichnungsmaterialien wurden wie nachstehend ausgewertet (Tabellen 3, 4 und 5).

(1) Dynamische Farbdichte:

Die Papiere (6 cm breite Streifen) wurden thermisch unter Verwendung des Atlantek 200 Testdruckers (Fa. Atlantek, USA) mit einer Kyocera-Druckleiste von 200 dpi und 560 Ohm bei einer angelegten Spannung von 20,6 V und einer maximalen Pulsbreite von 0,8 ms mit einem Schachbrett-Muster mit 10 Energieabstufungen bedruckt. Die Bilddichte (optische Dichte, o. D.) wurde mit einem einem SpectroEye-Densitometer von X-Rite bei einer Energiestufe von 0,45 mJ/Dot gemessen. Die Messunsicherheit der o.D.-Werte wird mit ≤2% veranschlagt.

(2) Statische Farbdichte (Starttemperatur):

Das Aufzeichnungsblatt wurde gegen eine Reihe auf unterschiedliche Temperaturen erhitzter und thermostatierter metallischer Stempel mit einem Anpressdruck von 0,2 kg/cm$^2$ und einer Kontaktzeit von 5 sec. gepresst (Thermoprüfer TP 3000QM, Maschinenfabrik Hans Rychiger AG, Steffisburg, Schweiz). Die Bilddichte (opt. Dichte) der so erzeugten Bilder wurde mit einem SpectroEye-Densitometer von X-Rite gemessen.

Der statische Startpunkt ist definitionsmäßig die niedrigste Temperatur, bei welcher eine optische Dichte von 0,2 erreicht wird. Die Genauigkeit des Messverfahrens ist $\leq\pm 0,5$ °C.

(3) Beständigkeitsprüfung des Druckbildes

a) Beständigkeit des Druckbildes unter den Bedingungen der künstlichen Alterung:

Je eine gemäß dem Verfahren von (1) dynamisch aufgezeichnete Probe des thermischen Aufzeichnungspapiers wurde für sieben Tage unter folgenden Bedingungen gelagert: i) 50 °C (Trocken-Alterung), ii) 40 °C, 85% relative Feuchte (Feucht-Alterung) und iii) unter Kunstlicht von Leuchtstoffröhren, Beleuchtungsstärke 16000 Lux (Licht-Alterung)
Nach Ablauf der Testzeit wurde die Bilddichte bei einer Bestromungsenergie von 0,45 mJ/dot gemessen und entsprechend der Formel (Gl. 1) in Bezug zu den entsprechenden Bilddichtewerten vor der künstlichen Alterung gesetzt.

$$\%\ verbleibende\ Bilddichte = \left(\frac{Bilddichte\ nach\ Test}{Bilddichte\ vor\ Test}\right) * 100 \qquad (\text{Gl. } 1)$$

Die Streuung der nach (Gl. 1) berechneten %-Werte beträgt $\leq\pm 2$ Prozentpunkte.

b) Weichmacherbeständigkeit:

Auf die gemäß dem Verfahren von (1) dynamisch aufgezeichnete Probe des thermischen Aufzeichnungspapiers wurde eine weichmacherhaltige
Frischhaltefolie (PVC-Folie mit 20 bis 25% Dioctyladipat) unter Vermeiden von Falten und Lufteinschlüssen in Kontakt gebracht, zu einer Rolle gewickelt und 16 Stunden gelagert. Eine Probe wurde bei Raumtemperatur (20 bis 22 °C), eine zweite bei 40 °C gelagert. Nach Abziehen der Folie wurde die Bilddichte (o.D.) gemessen und entsprechend der Formel (Gl. 1) in Bezug zu den entsprechenden Bilddichtewerten vor der Weichmacher-Einwirkung gesetzt.

c) Beständigkeit gegenüber Haftkleber:

Auf die gemäß dem Verfahren von (1) dynamisch aufgezeichnete Probe des thermischen Aufzeichnungspapiers wurde je ein Streifen transparentes Tesa-Selbstklebeband (tesafilm® kristall-klar, #57315) und getrennt davon ein Streifen Tesa-Verpackungsklebeband (#04204) unter Vermeiden von Falten und Lufteinschlüssen geklebt. Nach Lagerung bei Raumtemperatur (20 bis 22 °C) wurde nach 24 Stunden und nach 7 Tagen die Bilddichte (o.D.) - durch das jeweilige Klebeband hindurch - gemessen und entsprechend der Formel (Gl. 1) in Bezug zu den analog bestimmten Bilddichtewerten der frisch beklebten Muster gesetzt.

4) Lagerfähigkeit des unbedruckten Thermopapieres:

Ein Blatt Aufzeichnungspapier wurde in drei identische Streifen geschnitten. Ein Streifen wurde gemäß dem Verfahren von (1) dynamisch aufgezeichnet und die Bilddichte bestimmt. Die beiden anderen Streifen wurden im unbedruckten (weißen) Zustand für 4 Wochen in einem Klima von a) 40 °C und 85% relativer Feuchte (r. F.) und b) 60 °C und 50% relativer Feuchte (r. F.) gelagert.
Nach Klimatisierung der Papiere bei Raumtemperatur wurden diese gemäß dem Verfahren von (1) dynamisch bedruckt und die Bilddichte bei einer Bestromungsenergie von 0,45 mJ/dot mit dem Densitometer bestimmt. Die verbliebene Schreibleistung (%) der gelagerten zu den frischen (nicht gealterten) Mustern wurde gemäß der Gleichung (Gl. 1) berechnet.
Tabellen 3 bis 5 fassen die Auswertung der gefertigten Aufzeichnungsmaterialien zusammen.

Tabelle 3: Bilddichte, Starttemperatur und künstliche Alterung

| Farbentwickler | o.D. (0,45 mJ/dot) | Startpunkt (°C) | Künstliche Alterung* | | |
|---|---|---|---|---|---|
| | | | trocken | feucht | Licht |
| **III** | 1,21 | 75 | 97 | 98 | 87 |
| **IV** | 1,28 | 81 | 100 | 98 | 87 |
| **V** | 1,25 | 87 | 99 | 100 | 91 |

(fortgesetzt)

| Farbentwickler | o.D. (0,45 mJ/dot) | Startpunkt (°C) | Künstliche Alterung* | | |
|---|---|---|---|---|---|
| | | | trocken | feucht | Licht |
| **VII** | 1,32 | 76 | 100 | 100 | 86 |
| **VIII** | 1,30 | 83 | 100 | 100 | 88 |
| **X** | 1,21 | 79 | 100 | 100 | 80 |
| **XIV** | 1,29 | 104 | 99 | 99 | 91 |
| **XV** | 1,32 | 85 | 97 | 100 | 87 |
| **XVI** | 1,30 | 86 | 98 | 99 | 88 |
| **XVII** | 1,32 | 83 | 97 | 99 | 77 |
| **XVIII** | 1,28 | 76 | 99 | 100 | 81 |
| **XIX** | 1,35 | 87 | 98 | 98 | 85 |
| **XX** | 1,28 | 78 | 99 | 100 | 84 |
| **XXI** | 1,27 | 77 | 97 | 98 | 88 |
| **XXII** | 1,30 | 76 | 97 | 98 | 86 |
| Vergleichsbeispiel **NKK 1304** | 1,25 | 86 | 100 | 99 | 72 |
| Vergleichsbeispiel **PF 201** | 1,21 | 77 | 99 | 97 | 70 |
| *Prozentuale verbleibende Bilddichte entsprechend Gl. 1 | | | | | |

Tabelle 4: Beständigkeit des Druckbildes

| Farbentwickler | Tesa-Klebeband* | | | | Weichmacher-Folie* | |
|---|---|---|---|---|---|---|
| | 24 h | | 7 Tage | | 16 h | |
| | #57315 | #04204 | #57315 | #04204 | R.T. | 40 °C |
| **III** | 67 | 42 | 29 | 22 | 94 | 73 |
| **IV** | 73 | 53 | 49 | 32 | 99 | 92 |
| **V** | 78 | 61 | 57 | 43 | 100 | 94 |
| **VII** | 82 | 62 | 56 | 34 | 99 | 90 |
| **VIII** | 83 | 69 | 67 | 49 | 98 | 91 |
| **X** | 74 | 61 | 49 | 40 | 98 | 86 |
| **XIV** | 81 | 72 | 56 | 33 | 89 | 26 |
| **XV** | 83 | 73 | 57 | 42 | 97 | 90 |
| **XVI** | 73 | 57 | 43 | 36 | 98 | 86 |
| **XVII** | 89 | 92 | 78 | 77 | 94 | 94 |
| **XVIII** | 75 | 65 | 45 | 38 | 99 | 89 |
| **XIX** | 85 | 84 | 68 | 64 | 98 | 90 |
| **XX** | 81 | 76 | 58 | 54 | 97 | 92 |
| **XXI** | 80 | 73 | 56 | 53 | 98 | 91 |
| **XXII** | 84 | 80 | 63 | 64 | 98 | 91 |
| Vergleichsbeispiel **NKK 1304** | 40 | 18 | 10 | 10 | 76 | 15 |

(fortgesetzt)

| Farbentwickler | Tesa-Klebeband* | | | | Weichmacher-Folie* | |
|---|---|---|---|---|---|---|
| | 24 h | | 7 Tage | | 16 h | |
| | #57315 | #04204 | #57315 | #04204 | R.T. | 40 °C |
| Vergleichsbeispiel **PF 201** | 66 | 33 | 25 | 12 | 92 | 63 |
| *Prozentuale verbleibende Bilddichte entsprechend Gl 1. | | | | | | |

Tabelle 5: Schreibleistung nach Lagerung

| Farbentwickler | o.D. vor Lagerung | 4 Wochen 40 °C / 85% r. F. | | 4 Wochen 60 °C / 50% r. F. | |
|---|---|---|---|---|---|
| | | o.D. nach Lagerung | verbleibende o.D. * (%) | o.D. nach Lagerung | verbleibende o.D. * (%) |
| **III** | 1,21 | 1,21 | 100 | 1,19 | 98 |
| **IV** | 1,28 | 1,28 | 100 | 1,25 | 98 |
| **V** | 1,25 | 1,25 | 100 | 1,24 | 99 |
| **VII** | 1,32 | 1,31 | 99 | 1,28 | 97 |
| **VIII** | 1,30 | 1,29 | 99 | 1,29 | 99 |
| **X** | 1,21 | 1,21 | 100 | 1,12 | 93 |
| **XIV** | 1,29 | 1,29 | 100 | 1,29 | 100 |
| **XV** | 1,32 | 1,32 | 100 | 1,32 | 100 |
| **XVI** | 1,30 | 1,30 | 100 | 1,30 | 100 |
| **XVII** | 1,32 | 1,29 | 98 | 1,19 | 90 |
| **XVIII** | 1,28 | 1,28 | 100 | 1,23 | 96 |
| **XIX** | 1,35 | 1,33 | 99 | 1,31 | 97 |
| **XX** | 1,28 | 1,28 | 100 | 1,24 | 97 |
| **XXI** | 1,27 | 1,27 | 100 | 1,27 | 100 |
| **XXII** | 1,30 | 1,30 | 100 | 1,30 | 100 |
| Vergleichsbeispiel **NKK 1304** | 1,25 | 1,27 | 100 | 1,20 | 96 |
| Vergleichsbeispiel **PF 201** | 1,21 | 1,18 | 98 | 0,79 | 65 |
| *Prozentuale verbleibende Bilddichte entsprechend Gl. 1 | | | | | |

Den vorstehenden Beispielen lässt sich entnehmen, dass das wärmeempfindliche Aufzeichnungsmaterial der vorliegenden Erfindung insbesondere die folgenden vorteilhaften Eigenschaften zeigt:

(1) Das aufgezeichnete Bild der wärmeempfindlichen Aufzeichnungsmaterialien basierend auf den erfindungsgemäßen Farbentwicklern weist Druckdichten (optische Dichten) auf, die besser/vergleichbar jener der Vergleichsbeispiele mit bekannten Farbentwicklern sind (Tabelle 3).

(2) Die Temperatur, ab welcher eine visuell merkliche Vergrauung der erfindungsgemäßen Aufzeichnungsmaterialien eintritt (statischer Startpunkt), ist vergleichbar oder höher als bei den Vergleichsbeispielen mit bekannten Farbentwicklern (Tabelle 3).

(3) Die dem Alterungs-Test unterworfenen wärmeempfindlichen Aufzeichnungsmaterialien zeigen eine hohe Bildbeständigkeit, die besser oder vergleichbar zu den Vergleichsbeispielen mit bekannten Farbentwicklern ist (Tabelle 3).

(4) Das Druckbild ist nach Einwirkung hydrophober Agenzien (Klebstoffen, Weichmachern) kaum oder nur geringfügig verblasst. Die Bildbeständigkeit ist besser gegenüber wärmeemfindlichen Aufzeichnungsmaterialien mit bekannten nicht-phenolischen Farbentwicklern und erfüllt in hohem Maße die Anforderungen an markttaugliche wärmeempfindliche Aufzeichnungsmaterialien (Tabelle 4).

(5) Das Bedrucken der über mehrere Wochen unter extremen Bedingungen gelagerten wärmeempfindlichen Aufzeichnungsmaterialien führt zu Bilddichten, welche quasi identisch jenen der ungelagerten (frischen) wärmeempfindlichen Aufzeichnungsmaterialien sind (Tabelle 5).

(6) Mit den erfindungsgemäßen Farbentwicklern lässt sich ein in allen wichtigen anwendungstechnischen Belangen hochwertiges wärmeempfindliches Aufzeichnungsmaterial erhalten. Kein auf bekannten Farbentwicklern basierendes Aufzeichnungsmaterial weist ein vergleichbar gutes/ausgewogenes Leistungsprofil über alle Eigenschaften auf.

## Patentansprüche

**1.** Verbindung der Formel (I)

$$Ar^1\text{-NH-CO-NH-}C_6H_4\text{-}SO_2\text{-O-}C_6H_4\text{-NH-CO-NH-}Ar^2 \quad \text{(I),}$$

wobei $Ar^1$ und $Ar^2$ unabhängig voneinander ein unsubstituierter oder substituierter Phenyl-, Naphthyl- und/oder Heteroaryl-Rest sind.

**2.** Verbindung nach Anspruch 1, wobei $Ar^1$ ein Phenyl-Rest ist.

**3.** Verbindung nach Anspruch 1 oder 2, wobei $Ar^2$ ein Phenyl-Rest ist.

**4.** Verbindung nach mindestens einem der vorangegangenen Ansprüche, wobei $Ar^1$ mit mindestens einem $C_1$-$C_5$-Alkyl-, einem Alkenyl-, einem Alkinyl-, einem Benzyl-, einem Formyl-, einem Halogen-, einem NOz-, einem CN-, einem R-CO-, einem RO-, einem ROzC-, einem R-OCO-, einem R-$SO_2$-O-, einem R-O-$SO_2$-, einem R-$SO_2$-NH-, einem R-NH-$SO_2$-, einem R-NH-CO- oder einem R-CO-NH-Rest substituiert, wobei R ein $C_1$-$C_5$-Alkyl-, ein Alkenyl-, ein Alkinyl-, ein Phenyl-, ein Tolyl- oder ein Benzyl-Rest ist.

**5.** Verbindung nach mindestens einem der vorangegangenen Ansprüche, wobei $Ar^1$ einfach substituiert, vorzugsweise ein einfach substituierter Phenyl-Rest, ist.

**6.** Verbindung nach mindestens einem der vorangegangenen Ansprüche, wobei $Ar^2$ mit mindestens einem $C_1$-$C_5$-Alkyl-, einem Alkenyl-, einem Alkinyl-, einem Benzyl-, einem Formyl-, einem Halogen-, einem NOz-, einem CN-, einem R-CO-, einem RO-, einem $RO_2C$-, einem R-OCO-, einem R-$SO_2$-O-, einem R-O-$SO_2$-, einem R-$SO_2$-NH-, einem R-NH-$SO_2$-, einem R-NH-CO- oder einem R-CO-NH-Rest substituiert, wobei R ein $C_1$-$C_5$-Alkyl-, ein Alkenyl-, ein Alkinyl-, ein Phenyl-, ein Tolyl- oder ein Benzyl-Rest ist.

**7.** Verbindung nach mindestens einem der vorangegangenen Ansprüche, wobei $Ar^2$ einfach substituiert, vorzugsweise ein einfach substituierter Phenyl-Rest, ist.

**8.** Verbindung nach mindestens einem der vorangegangenen Ansprüche, wobei $Ar^1$ ein Phenyl-Rest und wobei $Ar^2$ ein Phenyl-Rest ist.

**9.** Verbindung nach mindestens einem der vorangegangenen Ansprüche, wobei der $Ar^1$-NH-CO-NH-Rest und der $Ar^2$-NH-CO-NH-Rest in 2- bzw. 3'-, in 2- bzw. 4'-, in 3- bzw. 2'-, in 3- bzw. 3'-, in 3- bzw. 4'-, in 4- bzw. 2'-, in 4- bzw. 3'- oder in 4- bzw. 4'-Stellung, bevorzugt in 3- bzw. 2'- oder 4-bzw. 3'-Stellung, zur -$C_6H_4$-$SO_2$-O-$C_6H_4$-Gruppe angeordnet sind.

**10.** Wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, wobei

der mindestens eine Farbentwickler die Verbindung der Formel (I) nach mindestens einem der Ansprüche 1 bis 9 ist.

**11.** Wärmeempfindliches Aufzeichnungsmaterial nach Anspruch 10, wobei der Farbentwickler in einer Menge von etwa 3 bis etwa 35 Gew.-%, bevorzugt von etwa 10 bis etwa 25 Gew.-%, bezogen auf den gesamten Feststoffgehalt der wärmeempfindlichen Schicht, vorliegt.

**12.** Wärmeempfindliches Aufzeichnungsmaterial nach Anspruch 10 oder 11, wobei der mindestens eine Farbbildner ein Farbstoff vom Triphenylmethan-Typ, vom Fluoran-Typ, vom Azaphthalid-Typ und/oder vom Fluoren-Typ, bevorzugt vom Fluoran-Typ, ist.

**13.** Wärmeempfindliches Aufzeichnungsmaterial nach mindestens einem der Ansprüche 10 bis 12, wobei neben dem phenolfreien Farbentwickler ein oder mehrere nicht-phenolische Farbentwickler vorliegen.

**14.** Verfahren zur Herstellung eines wärmeempfindlichen Aufzeichnungsmaterials nach mindestens einem der Ansprüche 10 bis 13, wobei auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-%, bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min, bevorzugt von mindestens etwa 1000 m/min, ganz besonders bevorzugt von mindestens etwa 1500 m/min, aufgetragen und getrocknet wird.

**15.** Wärmeempfindliches Aufzeichnungsmaterial, erhältlich gemäß dem Verfahren nach Anspruch 14.

## Claims

**1.** A compound of formula (I),

$$Ar^1\text{-NH-CO-NH-}C_6H_4\text{-}SO_2\text{-O-}C_6H_4\text{-NH-CO-NH-}Ar^2 \qquad (I),$$

wherein $Ar^1$ and $Ar^2$ independently of one another are an unsubstituted or substituted phenyl group, naphthyl group and/or heteroaryl group.

**2.** The compound according to claim 1, wherein $Ar^1$ is a phenyl group.

**3.** The compound according to claim 1 or claim 2, wherein $Ar^2$ is a phenyl group.

**4.** The compound according to at least one of the preceding claims, wherein $Ar^1$ is substituted with at least one $C_1$-$C_5$ alkyl group, an alkenyl group, an alkynyl group, a benzyl group, a formyl group, a halogen group, an NOz group, a CN group, an R-CO group, an RO group, an ROzC group, an R-OCO group, an R-SOzO group, an R-O-SOz group, an R-SOz-NH group, an R-NH-SOz group, an R-NH-CO group or an R-CO-NH group, wherein R is a $C_1$-$C_5$ alkyl group, an alkenyl group, an alkynyl group, a phenyl group, a tolyl group, or a benzyl group.

**5.** The compound according to at least one of the preceding claims,
wherein $Ar^1$ is substituted once, preferably is a phenyl group substituted once.

**6.** The compound according to at least one of the preceding claims, wherein $Ar^2$ is substituted with at least one $C_1$-$C_5$ alkyl group, an alkenyl group, an alkynyl group, a benzyl group, a formyl group, a halogen group, an NOz group, a CN group, an R-CO group, an RO group, an ROzC group, an R-OCO group, an R-SOzO group, an R-O-SOz group, an R-SOz-NH group, an R-NH-SOz group, an R-NH-CO group or an R-CO-NH group, wherein R is a $C_1$-$C_5$ alkyl group, an alkenyl group, an alkynyl group, a phenyl group, a tolyl group, or a benzyl group.

**7.** The compound according to at least one of the preceding claims, wherein $Ar^2$ is substituted once, preferably is a phenyl group substituted once.

**8.** The compound according to at least one of the preceding claims, wherein $Ar^1$ is a phenyl group, and wherein $Ar^2$ is a phenyl group.

**9.** The compound according to at least one of the preceding claims, wherein the $Ar^1$-NH-CO-NH group and the $Ar^2$-NH-CO-NH group are arranged respectively in the 2 and 3', in the 2 and 4', in the 3 and 2', in the 3 and 3', in the 3 and 4', in the 4 and 2', in the 4 and 3' or in the 4 and 4' position, preferably in the 3 and 2' or 4 and 3' position, relative to the - $C_6H_4$-$SO_2$-O-$C_6H_4$ group.

**10.** A heat-sensitive recording material comprising a carrier substrate and a heat-sensitive colour-forming layer, which contains at least one colour former and at least one phenol-free colour developer, wherein the at least one colour developer is the compound of formula (I) according to at least one of claims 1 to 9.

**11.** The heat-sensitive recording material according to claim 10, wherein the colour developer is present in an amount of from about 3 to about 35% by weight, preferably from about 10 to about 25% by weight, in relation to the total solids content of the heat-sensitive layer.

**12.** The heat-sensitive recording material according to claim 10 or claim 11, wherein the at least one colour former is a dye of the triphenylmethane type, of the fluoran type, of the azaphthalide type and/or of the fluorene type, preferably of the fluoran type.

**13.** The heat-sensitive recording material according to at least one of claims 10 to 12, wherein one or more non-phenolic colour developers are present in addition to the phenol-free colour developer.

**14.** A method for producing a heat-sensitive recording material according to at least one of claims 10 to 13, wherein an aqueous suspension containing the starting materials of the heat-sensitive colour-forming layer is applied to a carrier substrate and dried, wherein the aqueous application suspension has a solids content of from about 20 to about 75% by weight, preferably from about 30 to about 50% by weight, and is applied and dried by the curtain coating process at an operating speed of the coating plant of at least about 400 m/min, preferably at least about 1000 m/min, very especially preferably at least about 1500 m/min.

**15.** A heat-sensitive recording material obtainable by the method according to claim 14.

## Revendications

**1.** Composé de la formule (I)

$$Ar^1\text{-NH-CO-NH-}C_6H_4\text{-}SO_2\text{-O-}C_6H_4\text{-NH-CO-NH-}Ar^2 \qquad (I),$$

dans lequel $Ar^1$ et $Ar^2$ sont indépendamment l'un de l'autre un radical phényle, naphtyle et/ou hétéroaryle non substitué ou substitué.

**2.** Composé selon la revendication 1, dans lequel $Ar^1$ est un radical phényle.

**3.** Composé selon la revendication 1 ou 2, dans lequel $Ar^2$ est un radical phényle.

**4.** Composé selon au moins l'une quelconque des revendications précédentes, dans lequel $Ar^1$ est substitué par au moins un radical alkyle en $C_1$-$C_5$, un radical alcényle, un radical alcynyle, un radical benzyle, un radical formyle, un radical halogène, un radical $NO_2$-, un radical CN-, un radical R-CO-, un radical RO-, un radical $RO_2C$-, un radical R-OCO-, un radical R-$SO_2$-O-, un radical R-O-$SO_2$-, un radical R-$SO_2$-NH-, un radical R-NH-$SO_2$-, un radical R-NH-CO- ou un radical R-CO-NH, dans lequel R est un radical alkyle en $C_1$-$C_5$, un radical alcényle, un radical alcynyle, un radical phényle, un radical tolyle ou un radical benzyle.

**5.** Composé selon au moins l'une quelconque des revendications précédentes, dans lequel $Ar^1$ est un radical monosubstitué, de préférence un radical phényle monosubstitué.

**6.** Composé selon au moins l'une quelconque des revendications précédentes, dans lequel $Ar^2$ est substitué par au moins un radical alkyle en $C_1$-$C_5$, un radical alcényle, un radical alcynyle, un radical benzyle, un radical formyle, un radical halogène, un radical $NO_2$-, un radical CN-, un radical R-CO-, un radical RO-, un radical $RO_2C$-, un radical R-OCO-, un radical R-$SO_2$-O-, un radical R-O-$SO_2$-, un radical R-$SO_2$-NH-, un radical R-NH-$SO_2$-, un radical R-NH-CO- ou un radical R-CO-NH, dans lequel R est un radical alkyle en $C_1$-$C_5$, un radical alcényle, un radical alcynyle,

un radical phényle, un radical tolyle ou un radical benzyle.

7. Composé selon au moins l'une quelconque des revendications précédentes, dans lequel $Ar^2$ est monosubstitué, de préférence est un radical phényle monosubstitué.

8. Composé selon au moins l'une quelconque des revendications précédentes, dans lequel $Ar^1$ est un radical phényle et dans lequel $Ar^2$ est un radical phényle.

9. Composé selon au moins l'une quelconque des revendications précédentes, dans lequel le radical -NH-CO-NH $Ar^1$ et le radical -NH-CO-NH $Ar^2$ sont disposés en position 2 ou 3', en position 2 ou 4', en position 3 ou 2', en position 3 ou 3', en position 3 ou 4', en position 4 ou 2', en position 4 ou 3' ou en position 4 ou 4', de manière préférée en position 3 ou 2' ou en position 4 ou 3' par rapport au groupe $-C_6H_4-SO_2-O-C_6H_4$.

10. Matériau d'impression thermosensible comprenant un substrat de support ainsi qu'une couche chromogène thermosensible contenant au moins un agent chromogène et au moins un révélateur de couleur exempt de phénol, dans lequel l'au moins un révélateur de couleur est le composé de la formule (I) selon au moins l'une quelconque des revendications 1 à 9.

11. Matériau d'impression thermosensible selon la revendication 10, dans lequel le révélateur de couleur est présent en une quantité d'environ 3 à environ 35 % en poids, de manière préférée d'environ 10 à environ 25 % en poids, par rapport à la teneur totale en matières solides de la couche thermosensible.

12. Matériau d'impression thermosensible selon la revendication 10 ou 11, dans lequel l'au moins un agent chromogène est un colorant du type triphénylméthane, du type fluorane, du type azaphtalide et/ou du type fluorène, de manière préférée de type fluorane.

13. Matériau d'impression thermosensible selon au moins l'une quelconque des revendications 10 à 12, dans lequel un ou plusieurs révélateurs de couleur non phénoliques sont présents en parallèle du révélateur de couleur exempt de phénol.

14. Procédé de fabrication d'un matériau d'impression thermosensible selon au moins l'une quelconque des revendications 10 à 13, dans lequel une suspension aqueuse contenant les matériaux de départ de la couche chromogène thermosensible est appliquée sur un substrat de support et est séchée, dans lequel la suspension d'application aqueuse présente une teneur en matières solide d'environ 20 à environ 75 % en poids, de manière préférée d'environ 30 à environ 50 % en poids, et est appliquée avec le procédé de revêtement par couchage en rideau à une vitesse de fonctionnement de la coucheuse d'au moins environ 400 m/min, de manière préférée d'au moins environ 1000 m/min, de manière très particulièrement préférée d'au moins environ 1500 m/min et est séchée.

15. Matériau d'impression thermosensible pouvant être obtenu selon le procédé selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0526072 A1
- EP 0620122 B1
- WO 0035679 A1
- EP 2923851 A1
- WO 2017111032 A1
- JP H06227142 A
- EP 633145 A1
- JP H0821109 A
- JP H08244355 A
- JP H11268422 A
- DE 10196052 T1


### In der Beschreibung aufgeführte Nicht-Patentliteratur


- **M. ECKHARDT ; T.J. SIMAT.** *Chemosphere,* 2017, vol. 186, 1016